# EUROPEAN PATENT SPECIFICATION

(11) **EP 2 029 066 B1**
(45) Date of publication and mention of the grant of the patent: **16.03.2011**
(21) Application number: 06773678.5
(22) Date of filing: 20.06.2006
(51) Int. Cl.: A61F 2/92

(54) **SLIDE-AND-LOCK STENT**
SCHIEBE- UND ARRETIERSTENT
STENT A COULISSEMENT ET VERROUILLAGE

(43) Date of publication of application: 04.03.2009
(73) Proprietor: Reva Medical, Inc., San Diego, CA 92111 (US)
(72) Inventor: SCHMID, Eric V., San Diego, CA 92128 (US); MORRIS, Andrew, San Diego, CA 92126 (US)
(74) Representative: Straus, Alexander
(86) International application number: PCT/US2006/024127
(87) International publication number: WO 2007/149081

(56) References cited:
- EP-A- 0 756 853
- WO-A2-94/21196
- US-A- 5 618 299
- US-A1- 2006 020 324

## Description

### BACKGROUND OF THE INVENTION

### Field of the Invention

The invention relates to expandable medical implants for maintaining support of a body lumen.

### Description of the Related Art

Stents or expandable grafts are implanted in a variety of body lumens in an effort to maintain their patency. These devices are typically intraluminally implanted by use of a catheter, which is inserted at an easily accessible location and then advanced to the deployment site. The stent is initially in a radially compressed or collapsed state to enable it to be maneuvered through the lumen. Once in position, the stent is deployed which, depending on its configuration, may be achieved either automatically or manually, by for example, the inflation of a balloon about which the stent is carried on the catheter.

As stents are normally employed to hold open an otherwise blocked, constricted or occluded lumen, a stent must exhibit sufficient radial or hoop strength in its expanded state to effectively counter the anticipated forces. It is, however, simultaneously necessary for the stent to be as compact as possible in its collapsed state in order to facilitate its advancement through the lumen. As a result, it is advantageous for a stent to have as large an expansion ratio as possible.

An additional consideration is the longitudinal flexibility of the device. Such characteristic is important not only in maneuvering the stent into position, which may require the traversal of substantial convolutions of the vasculature, but also to better conform to any curvature of the vasculature at the deployment site. At the same time it is, however, necessary for the stent to nonetheless exhibit sufficient radial strength to provide the necessary support for the lumen walls upon deployment.

Another problem inherent in many prior art stent configurations is the longitudinal contraction that such structures typically undergo as they are radially expanded. This not only reduces the effective length of the stent in its deployed state but may cause abrasion trauma to be inflicted on the vessel walls during expansion.

A number of very different approaches have been previously devised in an effort to address these various requirements. A popular approach calls for the stent to be constructed wholly of wire. The wire is bent, woven and/or coiled to define a generally cylindrical structure in a configuration that has the ability to undergo radial expansion. The use of wire has a number of disadvantages associated therewith including for example, its substantially constant cross-section which may cause greater or lesser than an ideal amount of material to be concentrated at certain locations along the stent. Additionally, wire has limitations with respect to the shapes it can be formed into thus limiting the expansion ratio, coverage area, flexibility and strength that can ultimately be attained therewith.

As an alternative to wire-based structures, stents have been constructed from tube stock. By selectively removing material from such tubular starting material, a desired degree of flexibility and expandability can be imparted to the structure. Etching techniques as well as laser-cutting processes are utilized to remove material from the tube. Laser cutting provides for a high degree of precision and accuracy with which very well defined patterns of material can be removed from the tube to conversely leave very precisely and accurately defined patterns of material in tact. The performance of such stent is very much a function of the pattern of material which remains (i.e., design) and material thickness. The selection of a particular pattern has a profound effect on the coverage area, expansion ratio and strength of the resulting stent as well as its longitudinal flexibility and longitudinal dimensional stability during expansion.

While the tube-based stents offer many advantages over the wire-based designs, it is nonetheless desirable to improve upon such designs in an effort to further enhance longitudinal flexibility and longitudinal dimensional stability during radial expansion without sacrificing radial hoop strength.

One stent design described by Fordenbacher, see e.g., U.S. Patent Nos. 5,549,662 and 5,733,328, employs a plurality of elongated parallel stent components, each having a longitudinal backbone with a plurality of opposing circumferential elements or fingers. The circumferential elements from one stent component weave into paired slots in the longitudinal backbone of an adjacent stent component. By incorporating locking means with the slotted articulation, the Fordenbacher stent may minimize recoil after radial expansion. In addition, sufficient members of circumferential elements in the Fordenbacher stent may provide adequate scaffolding. Unfortunately the circumferential elements have free ends, protruding from the paired slots. Moreover, the circumferential elements weaving through the paired slots also necessarily stand off from the lumen wall. Both the free ends and the stand off may pose significant risks of thrombosis and/or restenosis. Moreover, this stent design would tend to be rather inflexible as a result of the plurality of longitudinal backbones.

Some stents employ "jelly roll" designs, wherein a sheet is rolled upon itself with a high degree of overlap in the collapsed state and a decreasing overlap as the stent unrolls to an expanded state. Examples of such designs are described in U.S. Patent Nos. 5,421,955 to Lau, 5,441,515 and 5,618,299 to Khosravi, and 5,443,500 to Sigwart. The disadvantage of these designs is that they tend to exhibit very poor longitudinal flexibility. In a modified design that exhibits improved longitudinal flexibility, multiple short rolls are coupled longitudinally. See e.g., U.S. Patent Nos. 5,649,977 to Campbell and 5,643,314 and 5,735,872 to Carpenter. However, these coupled rolls lack vessel support between adjacent rolls. Furthermore, these designs exhibit extensive overlapping of stent elements in multiple layers, which makes the delivery profile rather thick.

Various types of stents, including those referenced above, are often described based on their means for expansion. For additional information, a variety of stents types are described by Balcon et al., "Recommendations on Stent Manufacture, Implantation and Utilization," European Heart Journal (1997), vol. 18, pages 1536-1547, and Phillips, et al., "The Stenter's Notebook," Physician's Press (1998), Birmingham, Michigan.

Balloon expandable stents are manufactured in the collapsed condition and are expanded to a desired diameter with a balloon. The expandable stent structure may be held in the expanded condition by mechanical deformation of the stent as taught in, for example, U.S. Patent No. 4,733,665 to Palmaz. Alternatively, balloon expandable stents may be held in the expanded condition by engagement of the stent walls with respect to one another as disclosed in, for example, U.S. Patent Nos. 4,740,207 to Kreamer, 4,877,030 to Beck et al., and 5,007,926 to Derbyshire. Further still, the stent may be held in the expanded condition by one-way engagement of the stent walls together with tissue growth into the stent, as disclosed in U.S. Patent No. 5,059,211 to Stack et al.
WO 94/21196 refers to a multilayered percutaneously placeable stent comprising a sheet rolled in a spiral configuration, said sheet comprising multiple layers of biologically compatible materials of different physical properties. The stent may be locked in an open configuration by teeth provided at side edges of a sheet forming the tubular member.

Although balloon expandable stents are the first stent type to be widely used in clinical applications, it is well recognized that balloon expandable stents have a variety of shortcomings which may limit their effectiveness in many important applications. For example, balloon expandable stents often exhibit substantial recoil (i.e., a reduction in diameter) immediately following deflation of the inflatable balloon. Accordingly, it may be necessary to over-inflate the balloon during deployment of the stent to compensate for the subsequent recoil. This is disadvantageous because it has been found that over-inflation may damage the blood vessel. Furthermore, a deployed balloon expandable stent may exhibit chronic recoil over time, thereby reducing the patency of the lumen. Still further, balloon expandable stents often exhibit foreshortening (i.e., a reduction in length) during expansion, thereby creating undesirable stresses along the vessel wall and making stent placement less precise. Still further, many balloon expandable stents, such as the original Palmaz-Schatz stent and later variations, are configured with an expandable mesh having relatively jagged terminal prongs, which increases the risk of injury to the vessel, thrombosis and/or restenosis.

Self-expanding stents are manufactured with a diameter approximately equal to, or larger than, the vessel diameter and are collapsed and constrained at a smaller diameter for delivery to the treatment site. Self-expanding stents are commonly placed within a sheath or sleeve to constrain the stent in the collapsed condition during delivery. After the treatment site is reached, the constraint mechanism is removed and the stent self-expands to the expanded condition. Most commonly, self-expanding stents are made of Nitinol or other shape memory alloy. One of the first self-expanding stents used clinically is the braided "WallStent," as described in U.S. Patent No. 4,954,126 to Wallsten. Another example of a self-expanding stent is disclosed in U.S. Patent No. 5,192,307 to Wall wherein a stent-like prosthesis is formed of plastic or sheet metal that is expandable or contractible for placement.

Heat expandable stents are similar in nature to self-expanding stents. However, this type of stent utilizes the application of heat to produce expansion of the stent structure. Stents of this type may be formed of a shape memory alloy, such as Nitinol or other materials, such as polymers, that must go through a thermal transition to achieve a dimensional change. Heat expandable stents are often delivered to the affected area on a catheter capable of receiving a heated fluid. Heated saline or other fluid may be passed through the portion of the catheter on which the stent is located, thereby transferring heat to the stent and causing the stent to expand. However, heat expandable stents have not gained widespread popularity due to the complexity of the devices, unreliable expansion properties and difficulties in maintaining the stent in its expanded state. Still further, it has been found that the application of heat during stent deployment may damage the blood vessel.

In summary, although a wide variety of stents have been proposed over the years for maintaining the patency of a body lumen, none of the existing schemes has been capable of overcoming most or all of the above described shortcomings. As a result, clinicians are forced to weigh advantages against shortcomings when selecting a stent type to use in a particular application. Accordingly, there remains a need for an improved stent: one that is compact and flexible enough when collapsed to permit uncomplicated delivery to the affected area; one that is sufficiently flexible upon deployment to conform to the shape of the affected body lumen; one that expands uniformly to a desired diameter, without change in length; one that maintains the expanded size, without significant recoil; and one that has sufficient scaffolding to provide a clear through-lumen.

### SUMMARY OF THE INVENTION

For purposes of summarizing the invention, certain aspects, advantages and novel features of the invention have been described herein above. Of course, it is to be understood that not necessarily all such advantages may be achieved in accordance with any particular embodiment of the invention. Thus, the invention may be embodied or carried out in a manner that achieves or optimizes one advantage or group of advantages as taught or suggested herein without necessarily achieving other advantages as may be taught or suggested herein.

In one embodiment a slide-and-lock stent is disclosed. The stent comprises a tubular member having longitudinal and circumferential axes, said tubular member comprising: a first radial element comprising an elongate rail comprising a plurality of deflectable teeth, and a second radial element, circumferentially adjacent to the first radial element, and comprising an engagement means configured to slidably engage said elongate rail of the first radial element and deflect said deflectable tooth as the tooth contacts said engagement means; characterized in that said elongate rail defines upper and lower portions , said upper portion tapering from a first thickness to a second thickness along said circumferential axis approaching a distal edge of said upper portion, said lower portion tapering from said first thickness to said second thickness along said circumferential axis approaching a distal edge of said lower portion; and said engagement means comprising a closed loop that forms a slot, said closed loop tapering along said circumferential axis to said second thickness approaching a distal edge of said engagement means, said slot being sized and configured with said elongate rail being passable therethrough, such that said tubular member achieves expansion in the circumferential axis with reduced recoil.

According to an embodiment the elongate rail comprises two rail members with a gap being disposed therebetween.

According to another embodiment, the slot is sized and configured with the elongate rail being passable therethrough with the rail members the elongate rail deflecting toward one another into the gap when engaged by the slot.

According to still another embodiment, the engagement means comprise a closed loop which defines the slot.

According to an embodiment, the second thickness is approximately one-half of the first thickness.

According to another embodiment, the engagement means tapers from a combined thickness of approximately 1 ½ of the first thickness.

According to still another embodiment, the first and second radial elements have a combined maximum thickness of 1 ½ of the first thickness.

According to yet another embodiment,wherein the first thickness is approximately 0.1016 mm (0.0040 inches).

All of these embodiments are intended to be within the scope of the invention herein disclosed. These and other embodiments of the invention will become readily apparent to those skilled in the art from the following detailed description of the preferred embodiments having reference to the attached figures, the invention not being limited to any particular preferred embodiment(s) disclosed.

### BRIEF DESCRIPTION OF THE DRAWINGS

Having thus summarized the general nature of the invention and some of its features and advantages, certain preferred embodiments and modifications thereof will become apparent to those skilled in the art from the detailed description herein having reference to the figures that follow, of which:

**FIG.1** is a perspective partial view of a slide-and-lock stent in a partially expanded state.

**FIG. 2** is a perspective partial view of the stent of **FIG. 1** in a more expanded state.

**FIG. 3** is an enlarged planar view of a radial element of the stent of FIG. 1 illustrating a path of travel during deployment.

**FIG. 4** is a planar partial view of a module of a slide-and-lock stent, having passive radial elements with safety catches disposed in the longitudinal axis between each slide-and-lock radial element.

**FIG. 5** is a perspective partial view of a stent comprising the modules of **FIG. 4**, illustrating the operation of a safety catch mechanism.

**FIG. 6** is a planar partial view of a module having an actuating slide-and-lock radial element with a deflectable catch mechanism and positive return elements.

**FIG. 7** is a planar view of a module having actuating slide-and-lock radial elements with deflectable catch mechanisms similar to **FIG. 6**, but without any positive return elements.

**FIGS. 8** **and** **9** are planar partial views of an active lockout actuating slide-and-lock mechanism. **FIG. 8** shows the elements before actuation of the active lockout mechanism and **FIG. 9** shows the elements after actuation of the active lockout mechanism.

**FIGS. 10** **and** **11** are planar partial views of a deformable slide-and-lock stent and its operation having **FIG. 10** shows the deformable portion in a collapsed state. **Fig. 11** shows the deformable portion in an expanded state.

**FIG. 12** is a planar view of a module of the slide-and-lock stent incorporating deformable engaging tabs.

**FIG. 13** is a perspective partial view of a slide-and-lock stent incorporating intra-modular flexible elements having.

**FIG. 14** is a perspective partial view of a slide-and-lock stent incorporating intra-modular flexible elements.

**FIG. 15** is a planar partial view of a slide-and-lock stent incorporating intra-modular flexible elements and split deflectable rails.

**FIGS. 16** **and** **17** are a planar partial views of a slide-and-lock stent having a frangible deployment control mechanism incorporated into a passive radial element. **FIG.16** shows the deployment control mechanism before plastic deformation of the frangible members and **FIG. 17** shows the deployment control mechanism after plastic deformation of the frangible members.

**FIG. 18** is a simplified schematic view of a stent strut geometry configuration designed to create generally laminar flow conditions. .

**FIG. 19** is a simplified schematic view of another stent strut geometry configuration designed to create generally laminar flow conditions.

**FIG. 20** is a simplified schematic view of a differential thickness stent strut configuration designed to create generally laminar flow conditions, increase strength and reduce profile.

**FIG. 21** is a simplified schematic view of a tapered overlap stent wall configuration designed to create generally laminar flow conditions.

**FIG. 22** is a plan view illustrating yet another preferred embodiment of a row of radial elements wherein a solid wall is provided along a center portion.

**FIG. 23** illustrates a variation of the element of **FIG. 22** wherein an opening is provided along the center portion of the solid wall for providing fluid communication with a branch vessel.

**FIG. 24** illustrates another variation of an expandable stent wherein an expandable sheath is disposed over the expandable stent structure.

**FIG. 25** illustrates an alternative structure comprising deflectable teeth which deflect inward to provide a stent exhibiting mono-directional expansion.

**FIG. 26** illustrates another alternative structure comprising deflectable teeth which deflect downward to provide a stent exhibiting mono-directional expansion.

**FIG. 27** illustrates a portion of a single element from the drawing shown in **FIG. 26**.

**FIG. 28A** is a plan view illustrating an expandable stent comprising radial elements having deflectable teeth and a closed loop.

**FIG. 28B** illustrates the single module of **FIG. 28A** rolled into a partial tubular member.

**FIG. 28C** illustrates the articulation of two modules of **FIG. 28A** slidably interlocked to form a partial tubular member.

**FIG. 29** is a plan view illustrating a deflectable tooth module comprising circumferentially offset radial elements.

**FIG. 30A** is a plan view illustrating an expandable stent module comprising radial elements having deflectable teeth and a closed tapered loop corresponding to a tapered upper and lower portions of the radial element.

**FIG. 30B** illustrates the articulation of two modules of **FIG. 30A** slidably interlocked to form a tubular member.

**FIG. 30C** is an end view of the tubular member illustrated in **FIG. 30B** illustrating the interconnection of the closed tapered loop and the tapered upper and lower portions of the radial element.

**FIG. 31** illustrates another alternative structure comprising a single tab and series of shaped ridges that provide a stent exhibiting mono-directional expansion.

**FIG. 32** illustrates another alternative structure comprising a row of staggered radial elements that may be interconnected with similar structures to form an expandable stent.

**FIG. 33A** is a plan view illustrating slidably interconnected radial elements of the type illustrated in **FIG. 32** which are constrained in the collapsed condition.

**FIG. 33B** is a plan view illustrating slidably interconnected radial elements of the type illustrated in **FIG. 32** which are locked-out in the expanded condition.

**FIG. 34** is a perspective view illustrating another preferred embodiment of an expandable stent comprising a plurality of interconnected flexible rows.

**FIG. 34A** is a plan view illustrating a single flexible row from the stent embodiment **of** **FIG. 34****.**

**FIG. 35** is a perspective view illustrating yet another preferred embodiment of an expandable stent comprising a plurality of interconnected flexible rows.

**FIG. 35A** is a plan view illustrating a single flexible row from the stent embodiment of **FIG. 35****.**

**FIG. 36A** is a plan view illustrating another preferred embodiment of an expandable stent comprising a single element that may be rolled onto itself to form a tubular member.

**FIG. 36B** illustrates the single element of **FIG. 36A** rolled into a tubular member and constrained in the collapsed condition.

**FIG. 36C** illustrates the single element of **FIG. 36A** locked out in the expanded condition.

### DETAILED DESCRIPTION OF THE PREFERRED EMBODIMENT

The preferred embodiments of the invention described herein relate generally to expandable medical implant for maintaining support of a body lumen. Attributes of the stents include, but are not limited to, a non-actuating slide-and-lock stent with radial elements following a defined path geometry having both radial and axial translation; a slide-and-lock stent with longitudinal modules comprising both active (slide-and-lock) and passive radial elements wherein the radial elements have a variety of features including, but not limited to, spring elements, frangible deployment control mechanism and device overextension safety catches; a slide-and-lock stent with non-symmetric lockout geometries for enhanced sizing resolution; an actuating slide-and-lock stent with a positive lockout mechanism return; an actuating slide-and-lock stent with an active lockout system; a deformable slide-and-lock stent which provides additional device radial expansion and/or increases device safety, a slide-and-lock stent with two sided lockout features; a crimpable slide-and-lock stent for enhanced retention on a delivery balloon; a crush recoverable slide-and-lock stent; and a slide-and-lock stent with optimized strut or wall configuration to reduce turbulence and create generally laminar flow of the blood. Further embodiments include a slide-and-lock stent with a region with a high surface area region for support; a slide-and-lock stent with a region with a side-branch vessel access port; and, a slide-and-lock stent with a graft covering. Further embodiments include a slide-and-lock stent comprised of a biocompatible material (metal and/or polymer) and a slide-and-lock stent comprised of layered materials and/or spatially localized materials. Further embodiments include a expandable slide-and-lock stent module comprising radial elements having deflectable teeth and a closed loop wherein the radial elements of the stent are uniquely configured to include tapered sections in the circumferential direction in order to reduce the radial thickness of the stent at the overlapping sections of circumferentially adjacent stent modules.

While the description sets forth various embodiment specific details, it will be appreciated that the description is illustrative only and should not be construed in any way as limiting the invention. Furthermore, various applications of the invention, and modifications thereto, which may occur to those who are skilled in the art, are also encompassed by the general concepts described herein.

The term "stent" is used herein to designate embodiments for placement in (1) vascular body lumens (i.e., arteries and/or veins) such as coronary vessels, neurovascular vessels and peripheral vessels for instance renal, iliac, femoral, popliteal, subclavian and carotid; and in (2) nonvascular body lumens such as those treated currently i.e., digestive lumens (e.g., gastrointestinal, duodenum and esophagus, biliary ducts), respiratory lumens (e.g., tracheal and bronchial), and urinary lumens (e.g., urethra); (3) additionally such embodiments may be useful in lumens of other body systems such as the reproductive, endocrine, hematopoietic and/or the integumentary, musculoskeletal/orthopedic and nervous systems (including auditory and ophthalmic applications); and, (4) finally, stent embodiments may be useful for expanding an obstructed lumen and for inducing an obstruction (e.g., as in the case of aneurysms).

In the following description of the present invention, the term "stent" may be used interchangeably with the term "prosthesis" and should be interpreted broadly to include a wide variety of devices configured for supporting a segment of a body passageway. Furthermore, it should be understood that the term "body passageway" encompasses any lumen or duct within a body, such as those described herein.

Still further, it should be understood that the term "shape-memory material" is a broad term that includes a variety of known shape memory alloys, such as nickel-titanium alloys, as well as any other materials that return to a previously defined shape after undergoing substantial plastic deformation.

The assembled stent may generally comprise a tubular member having a length in the longitudinal axis and a diameter in the radial or circumferential axis sized for insertion into the body lumen. The tubular member is preferably formed with a "clear through-lumen," which is defined as having little or no structure protruding into the lumen in either the collapsed or expanded condition.

In many of the embodiments illustrated and described herein, the intraluminal stent is preferably provided with "slide-and-lock elements" generally referred to herein as "radial elements." The radial elements are slidably interconnected with circumferentially adjacent radial elements in a manner wherein the stent exhibits mono-directional radial expansion from a radially collapsed state to a radially expanded state, e.g., during deployment. The radial elements are preferably configured to provide a ratcheting effect such that the stent is maintained (i.e., "locked-out") in the expanded diameter after deployment within the body passage. More particularly, the structures (e.g., radial elements) may flex or bend; however, unlike conventional balloon expandable stents, no substantial plastic deformation of the elements are required during expansion of the stent from a collapsed diameter to an expanded diameter. Elements of this type are generally referred to herein as "non-deforming elements." Accordingly, the term "non-deforming element" is intended to generally describe a structure that substantially maintains its original dimensions (i.e., length and width) during deployment of the stent. Each radial element is preferably formed as a flat sheet that is cut or otherwise shaped to provide a slide-and-lock mechanism.

The term "radial strength," as used herein, describes the external pressure that a stent is able to withstand without incurring clinically significant damage. Due to their high radial strength, balloon expandable stents are commonly used in the coronary arteries to ensure patency of the vessel. During deployment in a body lumen, the inflation of the balloon can be regulated for expanding the stent to a particular desired diameter. Accordingly, balloon expandable stents may be used in applications wherein precise placement and sizing are important. Balloon expandable stents may be used for direct stenting applications, where there is no pre-dilation of the vessel before stent deployment, or in prosthetic applications, following a pre-dilation procedure (e.g., balloon angioplasty). During direct stenting, the expansion of the inflatable balloon dilates the vessel while also expanding the stent.

In another preferred embodiment, the stent further comprises a tubular member formed from a biocompatible and preferably, bioresorbable polymer, such as those disclosed in co-pending US Application No. 10/952,202. It is also understood that the various polymer formulae employed may include homopolymers and heteropolymers, which includes stereoisomers. Homopolymer is used herein to designate a polymer comprised of all the same type of monomers. Heteropolymer is used herein to designate a polymer comprised of two or more different types of monomer which is also called a co-polymer. A heteropolymer or co-polymer may be of a kind known as block, random and alternating. Further with respect to the presentation of the various polymer formulae, products according to embodiments of the present invention may be comprised of a homopolymer, heteropolymer and/or a blend of such polymers.

The term "bioresorbable" is used herein to designate polymers that undergo biodegradation (through the action of water and/or enzymes to be chemically degraded) and at least some of the degradation products are eliminated and/or absorbed by the body. The term "radiopaque" is used herein to designate an object or material comprising the
object visible by in vivo analysis techniques for imaging such as, but not limited to, methods such as x-ray radiography, fluoroscopy, other forms of radiation, MRI, electromagnetic energy, structural imaging (such as computed or computerized tomography), and functional imaging (such as ultrasonography). The term, "inherently radiopaque", is used herein to designate polymer that is intrinsically radiopaque due to the covalent bonding of halogen species to the polymer. Accordingly, the term does encompass a polymer which is simply blended with a halogenated species or other radiopacifying agents such as metals and their complexes.

In another preferred variation, the stent further comprises an amount of a therapeutic agent (for example, a pharmaceutical agent and/or a biologic agent) sufficient to exert a selected therapeutic effect. The term "pharmaceutical agent", as used herein, encompasses a substance intended for mitigation, treatment, or prevention of disease that stimulates a specific physiologic (metabolic) response. The term "biological agent", as used herein, encompasses any substance that possesses structural and/or functional activity in a biological system, including without limitation, organ, tissue or cell based derivatives, cells, viruses, vectors, nucleic acids (animal, plant, microbial, and viral) that are natural and recombinant and synthetic in origin and of any sequence and size, antibodies, polynucleotides, oligonucleotides, cDNA's, oncogenes, proteins, peptides, amino acids, lipoproteins, glycoproteins, lipids, carbohydrates, polysaccharides, lipids, liposomes, or other cellular components or organelles for instance receptors and ligands. Further the term "biological agent", as used herein, includes virus, serum, toxin, antitoxin, vaccine, blood, blood component or derivative, allergenic product, or analogous product, or arsphenamine or its derivatives (or any trivalent organic arsenic compound) applicable to the prevention, treatment, or cure of diseases or injuries of man (per Section 351 (a) of the Public Health Service Act (42 U.S.C. 262(a)). Further the term "biological agent" may include 1) "biomolecule", as used herein, encompassing a biologically active peptide, protein, carbohydrate, vitamin, lipid, or nucleic acid produced by and purified from naturally occurring or recombinant organisms, tissues or cell lines or synthetic analogs of such molecules, including antibodies, growth factors, interleukins and interferons; 2) "genetic material" as used herein, encompassing nucleic acid (either deoxyribonucleic acid (DNA) or ribonucleic acid (RNA), genetic element, gene, factor, allele, operon, structural gene, regulator gene, operator gene, gene complement, genome, genetic code, codon, anticodon, messenger RNA (mRNA), transfer RNA (tRNA), ribosomal extrachromosomal genetic element, plasmagene, plasmid, transposon, gene mutation, gene sequence, exon, intron, and, 3) "processed biologics", as used herein, such as cells, tissues or organs that have undergone manipulation. The therapeutic agent may also include vitamin or mineral substances or other natural elements.

In some embodiments, the design features of the radial elements can be varied to customize the functional features of strength, compliance, radius of curvature at deployment and expansion ratio. In some embodiments, the stent comprises a resorbable material and vanishes when its job is done. In some embodiments, the stent serves as a therapeutic delivery platform.

The stent preferably comprises at least one longitudinal module, which consists of a series of radial elements, including one or more slide-and-lock radial elements and optionally one or more passive radial elements, linked in the longitudinal axis by flexible coupling portions. Preferably, the radial elements from two or more similar longitudinal modules are slidably connected to circumferentially adjacent radial elements. Of course, single module (or jellyroll-type) embodiments are also encompassed within the scope of the present disclosure. Each module is preferably a discrete, unitary structure that does not stretch or otherwise exhibit any substantial permanent deformation during stent deployment.

Some embodiments relate to a radially expandable stent used to open, or to expand a targeted area in a body lumen. In some embodiments, the assembled stent comprises a tubular member having a length in the longitudinal axis and a diameter in the circumferential or radial axis, of appropriate size to be inserted into the body lumen. The length and diameter of the tubular member may vary considerably for deployment in different selected target lumens depending on the number and configuration of the structural components, described below. The tubular member is adjustable from at least a first collapsed diameter to at least a second expanded diameter. One or more stops and engaging elements or tabs are incorporated into the structural components of the tubular member whereby recoil (i.e., collapse from an expanded diameter to a more collapsed diameter) is minimized to less than about 5%.

The tubular member in accordance with some embodiments has a "clear through-lumen," which is defined as having no structural elements protruding into the lumen in either the collapsed or expanded diameters. Further, the tubular member has smooth marginal edges to minimize the trauma of edge effects. The tubular member is preferably thin-walled (wall thickness depending on the selected materials ranging from less than about 0.010 inches for plastic and degradable materials to less than about 0.002 inches for metal materials) and flexible (e.g., less than about 0.01 Newtons force/millimeter deflection) to facilitate delivery to small vessels and through tortuous vasculature.

Stents according to aspects of the present invention are preferably formed with walls for providing a low crossing profile and for allowing excellent longitudinal flexibility. In preferred embodiments, the wall thickness is about 0.00254 mm [0.0001] inches to about 0.635 mm [0.0250] inches, and more preferably about 0.0254 mm [0.00100] to about 0.254 mm [0.0100] inches. However, the wall thickness depends, at least in part, on the selected material. For example, the thickness may be less than about 0.1524 mm [0.0060] inches for plastic and degradable materials and may be less than about 0.0508 mm [0.0020] inches for metal materials. More particularly, for a 3.00 mm stent application, when a plastic material is used, the thickness is preferably in the range of about 0.1016 mn [0.0040] inches to about 0.1143 mm [0.0045] inches inches. However, a stent having various diameters may employ different thicknesses for biliary and other peripheral vascular applications. The above thickness ranges have been found to provide preferred characteristics through all aspects of the device including assembly and deployment. However, it will be appreciated that the above thickness ranges should not be limiting with respect to the scope of the invention and that the teachings of the present invention may be applied to devices having dimensions not discussed herein.

Some aspects of embodiments of stents are disclosed in U.S. Patent Nos. 6,033,436, 6,224,626 and 6,623,521. Some aspects are also disclosed in co-pending U.S. Patent Application Nos. 60/601,526, 10/655,338, 10/773,756, 10/897,235.

### Embodiments and Design Features of the Vascular Prosthesis

Preferred embodiments of a vascular prosthesis device or stent are disclosed herein. These embodiments teach unique design attributes and features that can be used in conjunction with a wide range of vascular prostheses or stents including embodiments of stents disclosed, taught or suggested herein, and/or prior art stents.

Preferred embodiments and additional design attributes and features allow for further improvement and optimization in vascular prosthesis devices or stents. The embodiments disclose novel geometries and mechanisms for vascular prosthesis devices or stents. These embodiments and attributes can be utilized individually or in combination to achieve desired optimum device performance and characteristics. Attributes of these embodiments are not limited to a particular material. Devices or attributes may be prepared from a variety of materials, including but not limited to, metals and polymers, including layers thereof, or any combination thereof, and any of the materials or combinations thereof disclosed, taught or suggested herein.

As used herein, one or more radial elements linked to one another in the longitudinal axis forms a module. The slidable interlocking of one or more modules in the circumferential axis forms a stent or vascular prosthesis. The stent is expandable via a sliding or articulating mechanism to allow variation in the stent diameter. The number of radial elements in a module and the number of modules comprising the stent can be efficaciously varied to provide for customization in the stent design and enhanced design versatility. Because longitudinally adjacent radial elements within a module are pre-linked (e.g., cut out of a single piece of material) in some preferred embodiments disclosed herein, there is no need to weld and/or otherwise connect the radial elements within a module. Likewise, radial elements from circumferentially adjacent modules are preferably interlinked (e.g., via insertion of tabs or rails within slots) during assembly without any welding and/or other fixed connections.

As detailed herein, various methods and techniques may be used to fabricate or manufacture the stents of embodiments of the invention. These include injection molding, laser machining, laser cutting, laser ablation, die-cutting, chemical etching, plasma etching or other methods known in the art which are capable of producing high-resolution components. In some embodiments, the stent is fabricated from a biodegradable material.

The stents and prostheses of embodiments of the invention can have many application and can be utilized in various techniques and in combination with other procedures; some of which are disclosed herein. One use of the stents is coronary stenting applications. The stenting can be performed in conjunction with other catheter-based procedures, such as balloon angioplasty or artherectomy. The stents typically allow for an excellent final result to be obtained with little to no narrowing remaining within the coronary arteries. By performing a stent insertion along with other procedures, such as balloon angioplasty or artherectomy, the risk of the artery re-closing (restenosis) is greatly reduced.

Various polymer materials may be used in conjunction with the stents, as described herein such as in the sections "Polymeric Stents" and "Differential Layered and Spatially Localized Vascular Prosthesis." Various therapeutic agents may also be incorporated into the stents as described as in these sections.

If desired for a particular application, embodiments of the present invention may be used with a delivery sheath to constrain the stent in the collapsed condition and to protect the inner wall of the vessel during stent delivery. For example, a retractable delivery sheath may be configured for enclosing the stent during delivery. After the treatment site is reached, the sheath is withdrawn to expose the stent.

In an alternative configuration, the stents may be used in combination with a covering or sheath to provide a vessel graft. Different regions of the stent may exhibit different expanded diameter, and the actual number and dimensions of the radial elements may vary. The locking mechanism may also be releasable.

It will be appreciated by those skilled in the art that the basic design of a series of slide-and-lock radial elements provides the manufacturer with a great deal of flexibility with regard to the collapsed and expanded diameters of the stent as well as the longitudinal length. Increased expanded diameter and expansion ratio can be achieved by increasing the number of modules, e.g., the number of slidably interconnected radial elements that comprise the circumference of the tubular member. Increased longitudinal length can be achieved by increasing the number of radial elements within a module.

In another variation of the embodiments of the stent, different regions within the stent may exhibit different expanded diameters, such that the stent may be adjustable to different luminal states along the length of the stent. Accordingly, the stent may exhibit a tapered configuration in its deployed state, having a larger diameter at one end with progressive or step-wise decreases in expanded diameter moving toward the other end of the stent.

It will be appreciated by those of skill in the art that the interlocking and sliding radial element design of embodiments of the invention provides the manufacturer with substantial flexibility in customizing the stent for different applications. Because overlap of stent components is minimized by the nesting frame elements, the collapsed profile can be very thin without compromising radial strength. Moreover, the degree of overlap does not change substantially during expansion, unlike jelly-roll designs which expand by unraveling of a rolled sheet. Furthermore, the deployment flexibility of the stent of embodiments of the invention can be customized by changing the length, configuration and number of radial elements employed. Thus, a very flexible and ultra-thin embodiment of the stent is deemed to be uniquely suited for deployment in small and difficult to reach vessels, such as the intercranial vessels distal to the carotids and the remote coronary vessels.

The construction of the stent in this fashion provides a great deal of benefit over the prior art. The construction of the locking mechanism is largely material-independent. This allows the structure of the stent to comprise high strength materials, not possible with designs that require deformation of the material to complete the locking mechanism. The incorporation of these materials will allow the thickness required of the material to decrease, while retaining the strength characteristics of thicker stents. In preferred embodiments, the frequency and arrangement of locking holes, stops or teeth present on selected elements prevents unnecessary recoil of the stent subsequent to expansion.

In any of the embodiments taught or suggested herein, materials may be used that exhibit clinical visibility (radiopacity), e.g., by incorporation of iodine or bromine or other radiopaque elements, use of iodine-containing or other contrast agents. Materials may be non-resorbable polymers or radiopaque constituents of metal particulates, bands or even liquid gold. Methods for viewing may include, but are not limited to, x-ray, fluoroscopy, ultrasound, MRI, or Imatron Electron Beam Tomography (EBT).

### Non-Actuating Slide-and-lock Device Design

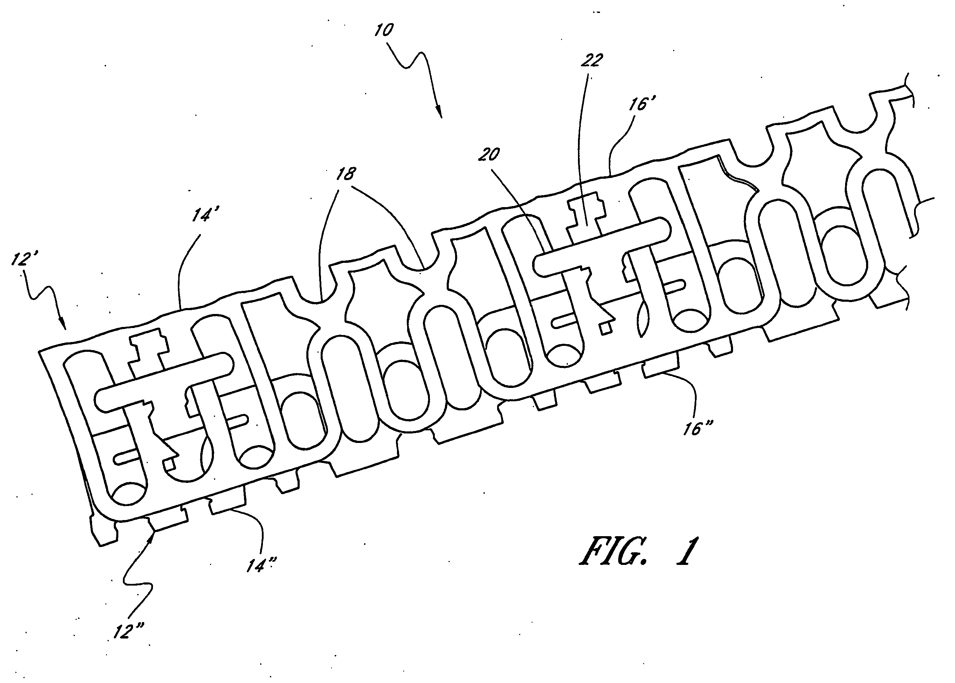

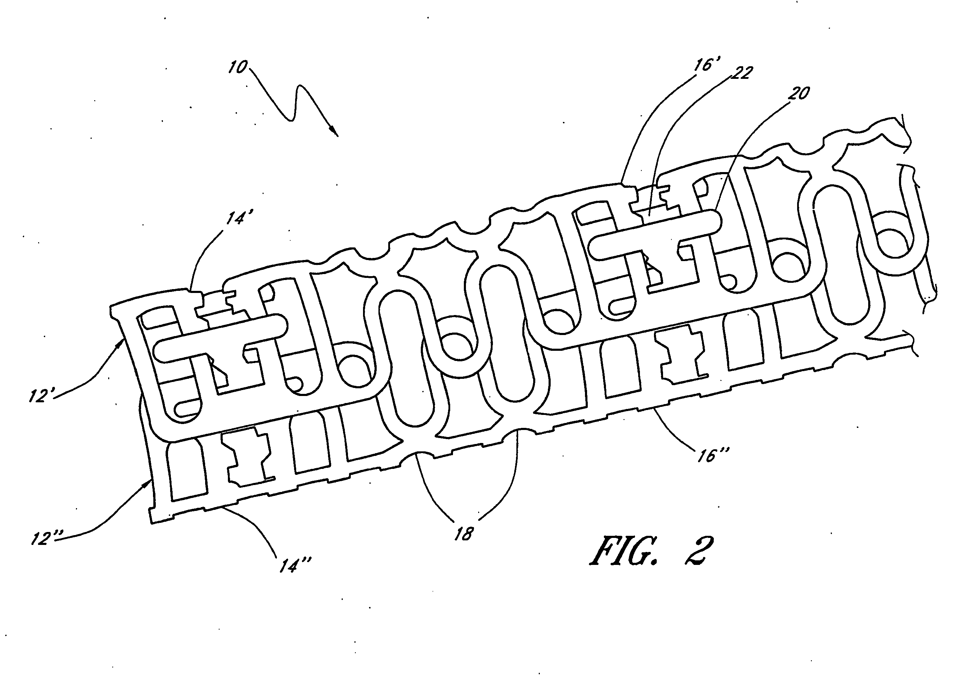

**FIGS. 1-3** show partial views of a slide-and-lock stent or vascular prosthesis device 10. **FIG. 1** shows the stent 10 in a partially-expanded state and **FIG. 2** shows the stent **10** in an expanded state.

The embodiment illustrated in **FIGS. 1-3** is a slide-and-lock stent device 10 that employs no actuating (that is, flexing, bending, and the like) elements to achieve expansion and lockout.

**FIGS. 1** **and** **2** show partial views of two circumferentially adjacent modules 12' and 12", each having longitudinally offset slide-and-lock radial elements, 14' and 16' in module 12', and 14" and 16" in module 12". Modules generally have at least two (2) slide-and-lock radial elements, at proximal and distal ends of the module. These are sometimes referred to as mechanism radial elements, because they comprise the slide:-and-lock mechanisms that provide controlled deployment and resist radial compression. In preferred embodiments of these modules, there are between 2 and 8 slide-and-lock radial elements, and more preferably, between 2 and 4 slide-and-lock radial elements per module.

In some embodiments, such as that illustrated in **FIGS. 1-3****,** the longitudinally offset slide-and-lock radial elements within a module are separated and interconnected by one or more passive radial elements such as the two (2) passive radial elements 18 shown in **FIGS. 1** **and** **2****.** These passive radial elements are sometimes referred to as non-mechanism radial elements because they do not contribute to the slide-and-lock mechanism of radial expansion, like the slide-and-lock radial elements. In some embodiments, there are no passive radial elements. In other embodiments, there are from 1 to 8 passive radial elements disposed between each slide-and-lock radial element. More preferably, there are from 1 to 4 passive radial elements disposed between each slide-and-lock radial element in a module. As disclosed in greater detail below, these passive, non-mechanism radial elements can be engineered in many different geometric configurations to provide *inter alia* variable flexibility, variable radial strength, variable scaffolding (vessel wall coverage), and/or a safety catch to prevent over-expansion.

As can be seen in **FIGS. 1** **and** **2****,** a tab 20 on each slide-and-lock radial element (shown here on 16") is slidably engaged within a slot 22 in the circumferentially adjacent slide-and-lock radial element (shown here in 16'). The entire circumference of stent 10 may comprise from 1 to 8 circumferentially adjacent modules, more preferably from 2 to 6 circumferentially adjacent radial elements, and most preferably from 2 to 4 circumferentially adjacent radial elements.

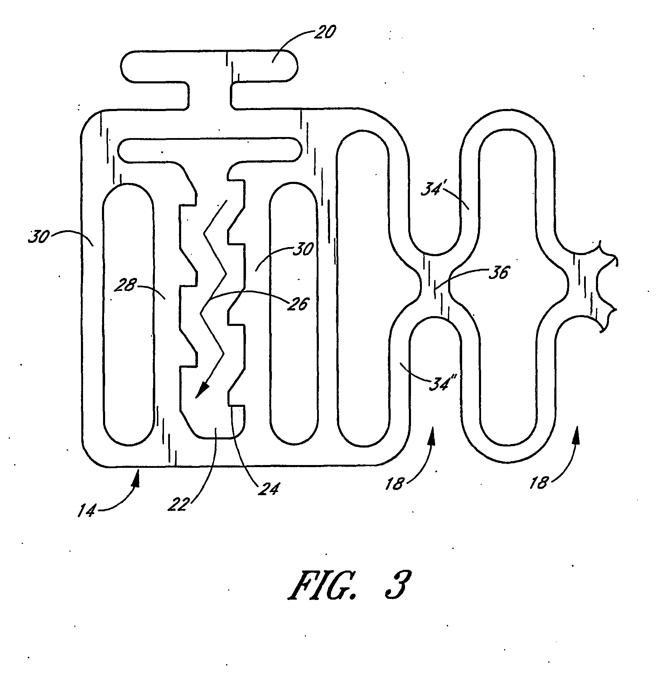

As best seen in **FIG. 3****,** a slide-and-lock radial element 14 has a slot 22 with lockout teeth, catches or stops 24. When a tab 20 is slidably engaged within a slot 22 from a circumferentially adjacent slide-and-lock radial element, it can travel within the slot 22- thereby traveling through a defined travel path, as generally indicated by arrow 26 in **FIG. 3****.** The travel path may be disposed substantially in the circumferential axis as shown, or in some embodiments, the travel path may traverse both circumferential and longitudinal axes. Advantageously, the slot 22, stop 24, and tab 20 configurations allow for expansion that achieves radial expansion while restricting travel in the opposite direction. Defined path geometry can easily be altered to achieve a variety of device performance attributes, for example, lower/higher deployment pressures and the like, among others.

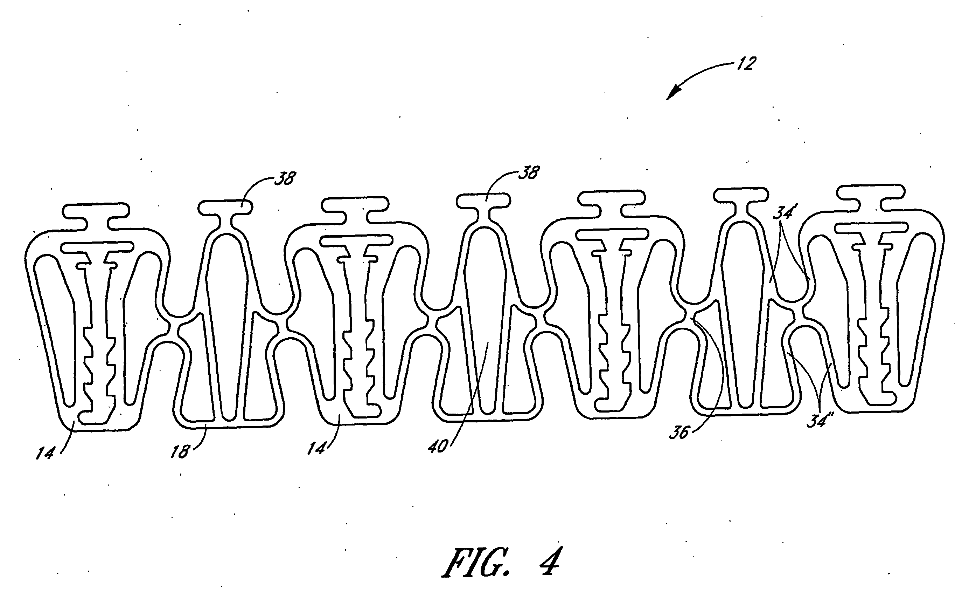

In the illustrated embodiment of the slot 22 shown in **FIG. 3****,** the stops 24 are circumferentially offset from one another and disposed on alternating proximal 28 and distal 30 sides or walls of the slot. Further, the illustrated catches 24 are configured so as to allow the tab 20 to slide past each stop, translating simultaneously in the longitudinal (axial) and circumferential (radial) axes, while moving along the travel path 26. However, the stop 24 is configured to prevent the tab 20 from moving backwards along the travel path 26. The slide-and-lock mechanism illustrated in **FIGS. 1-3** does not involve material bending or deformation of the stent materials. Of course, other slot 22, stop 24, and tab 20 configurations are encompassed, as long as they facilitate one-way sliding of the tab 20 within the slot 22. Further examples of different slot and stop configurations are disclosed with reference to **FIGS. 4-16****.** The configurations disclosed herein are generally designed to allow one-way sliding to a more expanded circumference, while preventing significant recoil.

In the illustrated embodiment (**FIGS. 1-3**), there are also frame elements 32 (shown in **FIG. 3**), which surround the radial element 14. In some preferred embodiments, there are no frame elements. In others, as shown, the frame elements may be used to provide additional scaffolding and/or radial strength.

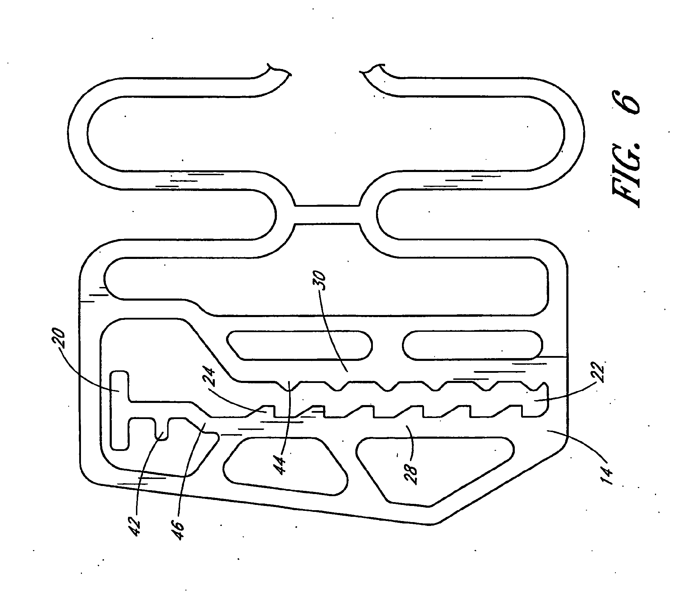

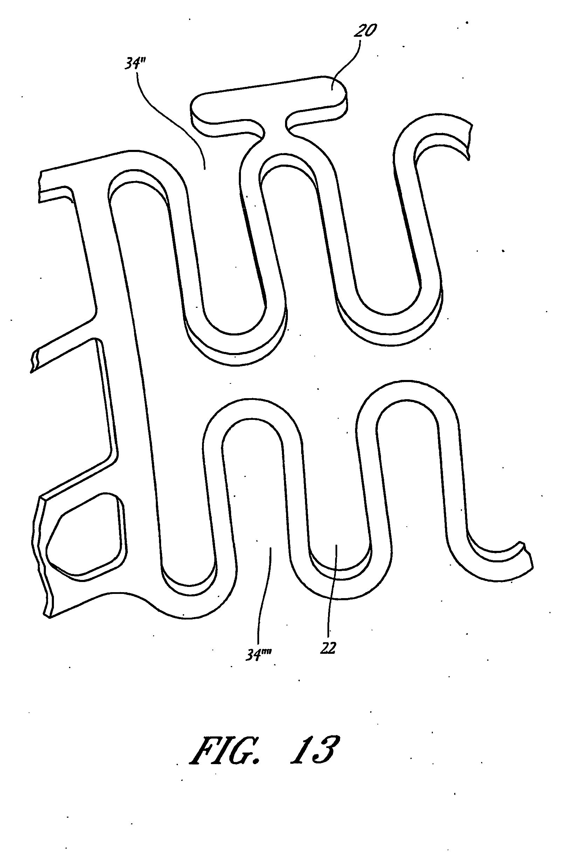

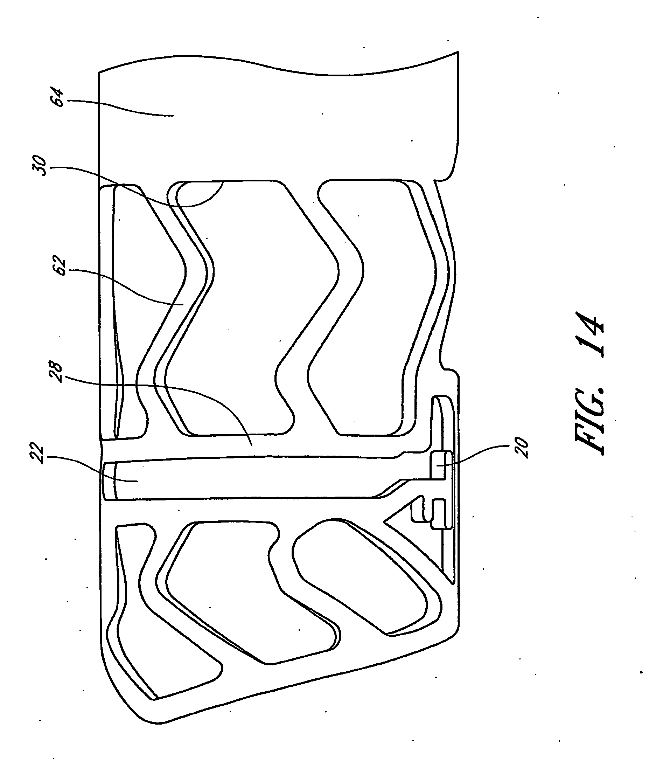

The passive radial elements 18 illustrated in **FIG. 3** comprise U-shaped members 34' and 34", which are inverted with respect to one another. The apices of the inverted U-shaped members are connected to one another by a linkage element 36. The configurations of the passive radial elements may vary greatly depending on the desired stent attributes. For example, the inverted U-shaped members 34' and 34" and the linkage element 36 of a passive radial element may be aligned within the module in a direction substantially parallel to the circumferential axis (as shown in **FIGS. 1-3**). Alternatively, the inverted U-shaped members 34' and 34" and the linkage element 36 of a passive radial element may be aligned within the module in a direction which is diagonal to the circumferential axis (as shown for example in **FIGS. 4-6**). In other variations, the linkage element 36 which connects the apices of inverted U-shaped members 34' and 34" may be short (as shown in **FIG. 3**) or relatively much longer (as shown for example in **FIGS. 6-7**). The linkage element 36 may also be configured to enhance flexibility, e.g., in a serpentine or spring-shape. In some preferred embodiments, the passive radial elements may not include U-shaped members at all. Instead, a variety of passive, non-mechanism radial element configurations may be employed between slide-and-lock radial elements. Some examples are shown in **FIGS. 13** **and** **14****.**

In preferred embodiments, each module is formed from a single piece of material-thereby avoiding any welding or other connections between longitudinally adjacent mechanism and non-mechanism radial elements. Alternatively, the slide-and-lock and passive radial elements within a longitudinal module may be attached to one another by a weldless connection, e.g., adhesive. Welded connections are also encompassed within the present disclosure. Further details of the stent construction are provided below in the Sections entitled "Metal Stents," "Polymeric Stents" and "Methods of Manufacturing and Assembling Polymeric Stents."

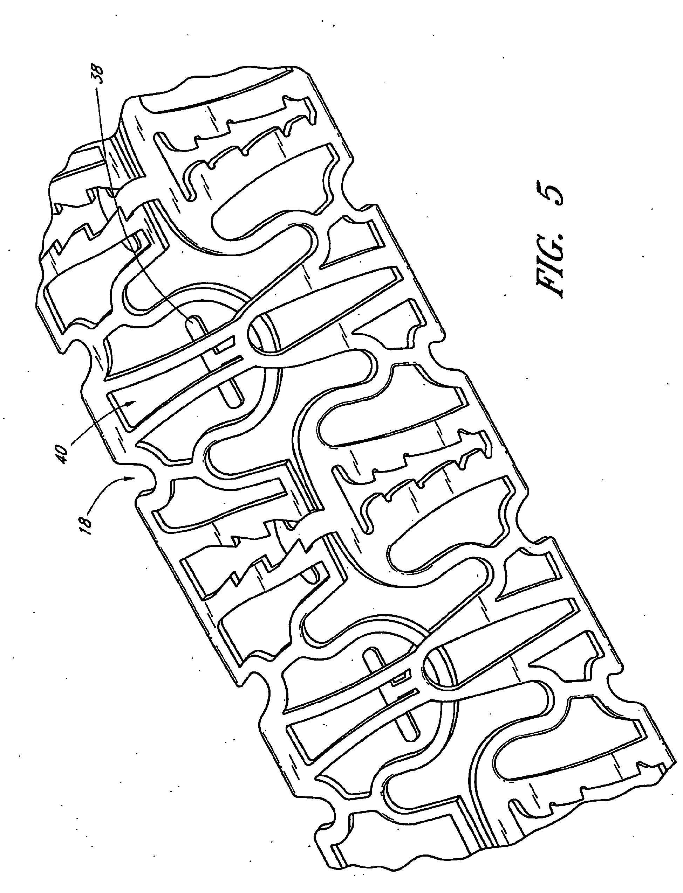

**FIG. 4** shows a longitudinal module 12 of a slide-and-lock stent or vascular prosthesis device. Modular device designs allow for a wide variety of combinations of mechanism and non-mechanism module components. As can be seen generally from **FIG. 4****,** there are alternating mechanism and passive (or non-mechanism) radial elements, with one passive radial element 18 disposed between each slide-and-lock radial element 14 in the module 12; although other configurations can be substituted with efficacy as needed or desired. Where N = the number of radial elements in a module, then up to N-1 passive radial elements may be employed. More preferably, a module has at least two slide-and-lock radial elements, wherein at least N-2 passive radial elements may be used. The passive radial elements in this embodiment have safety catches or tabs 38 and slots 40, but the slots do not have any stops, teeth, catches or other lockout structures. Accordingly, as illustrated in **FIG. 5**, when a safety tab 38 from one passive radial element is slidably engaged in the slot 40 of a circumferentially adjacent passive radial element, there is neither resistance to expansion during deployment nor resistance to recoil; thus, the radial element is still referred to as a passive or non-mechanism radial element However, when the safety tab 38 engaged in the slot 40 slides during deployment (radial expansion) to the end of the slot 40, it will prevent further expansion during deployment, thereby providing a safety mechanism against over-expansion. As discussed above, the passive radial elements can be designed to provide a variety of features and characteristics, including, but not limited to, providing enhanced flexibility such as with spring elements (discussed further below), deployment mechanism control elements (discussed further below), preferential side branch access locations or points, and device over-extension safety catches (as discussed with regard to the safety tabs 38 and slots 40 shown in **FIGS. 4** **and** **5****.**

One important aspect of a slide-and-lock design is the sizing resolution achievable during deployment, and the amount of recoil exhibited during compressive loading. The finer the mechanism of the design, the higher the sizing resolution and the lower recoil exhibited. One embodiment of the slide-and-lock radial element that facilitates sizing resolution and recoil resistance employs staggered non-symmetric lockout geometries. **FIGS. 1-5** illustrate examples of such staggered non-symmetric lockout geometry, wherein the slots 22 have stops 24 arranged in a staggered pattern, on both proximal 28 and distal 30 sides of the slot 22.

### Actuating Slide-and-lock Design

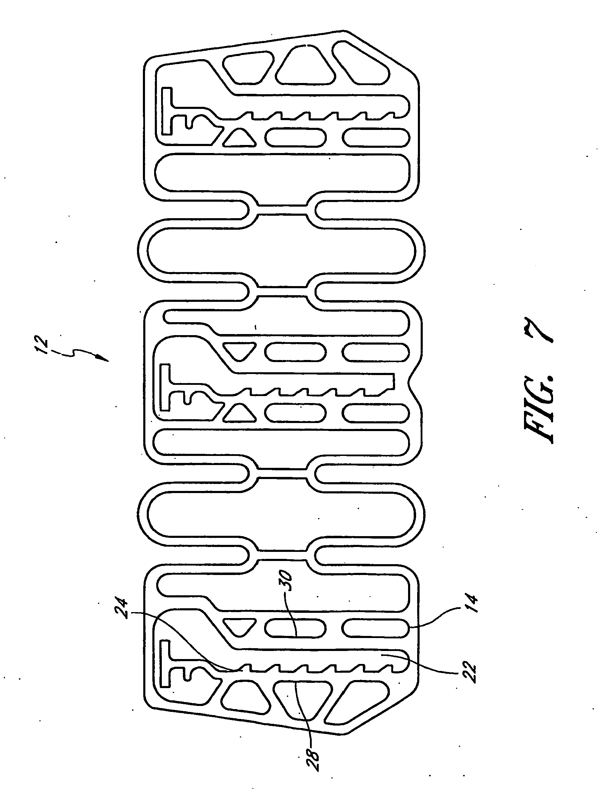

In an alternate embodiment, the slide-and-lock radial elements may employ different non-symmetric lockout geometry, wherein all of the stops 24 are located on only one side of the slot 22 (See e.g., **FIGS. 6** **and** **7****).** Of course, those skilled in the art will appreciate that regardless of whether a staggered or one-sided stop configuration is employed, one can vary the sizing resolution by varying the number of stops and the distance between individual stops, such that as the distance between stops becomes lesser, sizing resolution increases, and as the distance between stops becomes greater, sizing resolution decreases. With reference to **FIG. 6****,** the slide-and-lock radial element 14 has a tab 20, an actuating catch member 42, and a slot 22. All of the stops 24 are located on one side of the slot-on the proximal side 28 in the illustrated embodiment. Positive return elements 44 are located on the opposite side of the slot-on the distal side 30 in the illustrated embodiment. While the tab 20 substantially maintains travel within the radial axis, the catch member 42 is disposed along a flexible neck 46, such that as the tab 20 slides through slot 22, the interaction of the catch member 42 with the stops 24 causes the neck 46 to deflect toward the distal side 30. In the illustrated embodiment of **FIG. 6****,** to further optimize the performance of an actuating slide-and-lock mechanism, a positive return element 44 is included into the deployment mechanism to ensure return of the deflected neck 46 and the catch member 42 to its non-actuated position. More particularly, during radial expansion, the catch mechanism, including the tab 20 and catch member 42, is first deflected distally (actuated) by the lockout stop 24. Once the catch member 42 has passed the stop 24, it can either elastically return to its natural position (discussed further below) or, as illustrated in **FIG. 6****,** the positive return element 44 is employed to redirect the catch mechanism (tab 20 and catch member 42) to its natural, pre-actuated position within the slot 22, such that the catch member 42 catches, engages, or is otherwise prevented by its interaction with the lockout stop 24, from moving backwards to a more collapsed state (recoil).

**FIG. 7** shows a module 12 employing an actuating slide-and-lock radial element 14, similar to that shown and described with reference to **FIG. 6****.** In the illustrated embodiment of **FIG. 7**, all of the stops 24 are located on the proximal side 28 of the slot 22, like the embodiment shown in **FIG. 6****;** however, there are no positive return elements (44 in **FIG. 6**) located along the distal side 30 of the slot 22 in **FIG. 7****.** Instead, in this embodiment, the catch mechanism is designed to elastically return to its natural pre-actuated position.

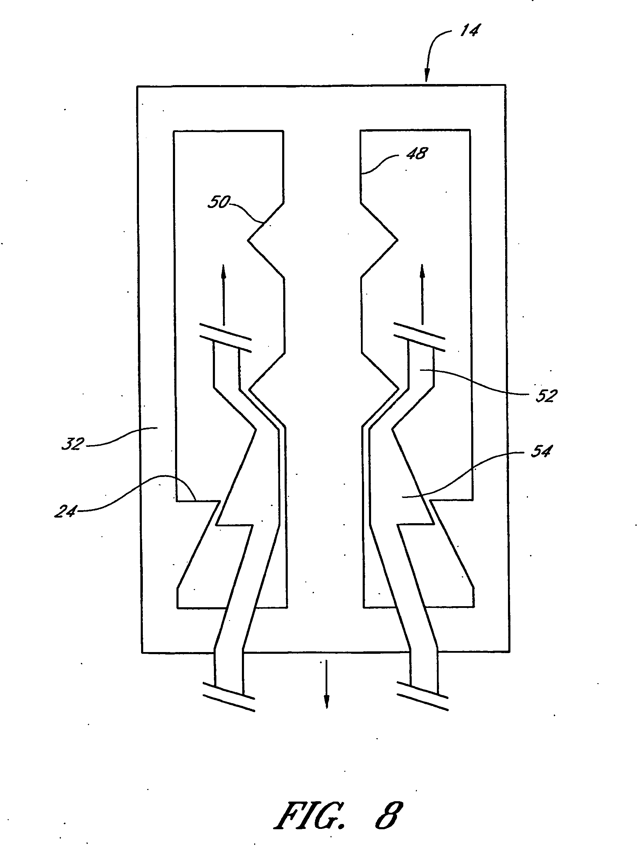

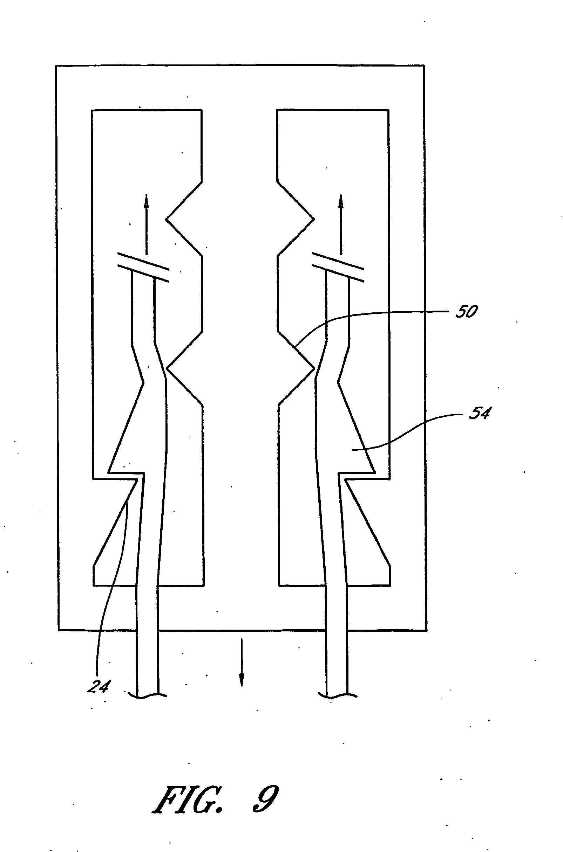

**FIGS. 8** **and** **9** are planar partial views of an active lockout mechanism, wherein a deflectable element is actively positioned by another feature/geometry of the design to engage the lockout mechanism. Here with reference to **FIGS. 8** **and** **9****,** the drawings show a partial slide-and-lock radial element 14, comprising a non-deflectable actuating rail 48 (which is centrally disposed in the illustrated embodiment), having disposed thereon an actuator 50, a plurality of which are shown symmetrically disposed along both proximal and distal surfaces of the central rail in the illustrated embodiment. Slidably engaged with the actuating rail 48 is a deflectable rail 52, comprising a deflectable catch element 54; two catch elements 54 are shown symmetrically disposed along the deflectable rail 52 in the illustrated embodiment. The actuators 50 on the actuator rail 48 and deflectable catch elements 54 on the deflectable rail 52 are configured so that as the deflectable rail 52 slides along the actuating rail 48, the actuators 50 cause the deflectable catch elements 54 to deflect outward. This active lockout mechanism also includes teeth or stops 24 (disposed along a frame element 32 in the illustrated embodiment), which are adapted to engage the deflectable catch elements 54 once actuated, thereby preventing radial recoil. **FIG. 8** shows the radial element before sliding causes actuation of the active lockout mechanism. **FIG. 9** shows the radial element after actuation of the active lockout mechanism, wherein the deflectable catch elements 54 are shown deflected outward by the actuators 50 and engaging the stops 24.

In variations to the illustrated embodiment, the actuators 50, deflectable catch elements 54, and stops 24, may be positioned on any of the components of the slide-and-lock radial element, as long as the actuators 50 are positioned to cause active deflection of the slidably engaged deflectable catch elements 54, such that deflection results in engagement of the stops 24 and lockout (inhibition of radial recoil).

### Deformable Slide-and-lock Stent

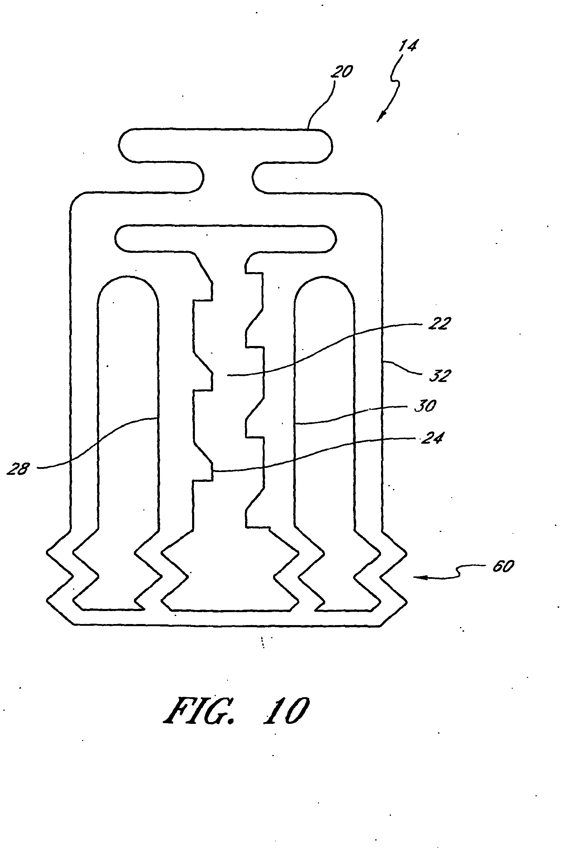

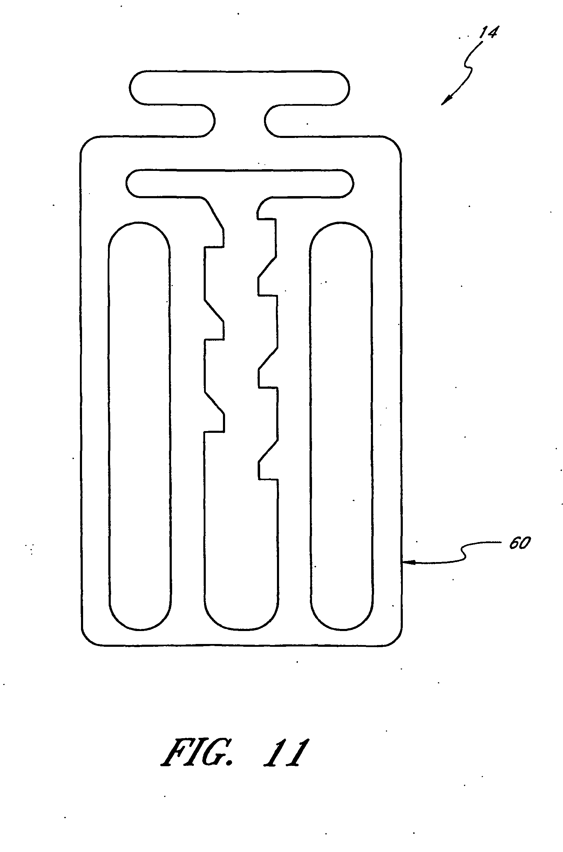

**FIGS. 10** **and** **11** are plan views of a slide-and-lock radial element. The slide-and-lock radial element 14 illustrated in **FIG. 10** has a tab 20, slot 22, stops or teeth 24, and a frame element 32 similar to those shown in **FIG. 3****.** However, the slide-and-lock radial element 14 also comprises a deformable region 60. In the illustrated embodiment, the proximal and distal portions of the frame element 32 as well as the proximal 28 and distal 30 portions of the slot wall are modified in the deformable region 60 to allow expansion and/or contraction in the radial axis through material deformation. Of course, those skilled in the art will readily appreciate that a variety of material configurations, including for example, zig-zag, U-shaped, serpentine, waves, undulating, and angled configurations, as well as changes in material cross-section (e.g., from a flat sheet to a bendable wire), can be employed so as to produce regions of deformability. Lengthwise adjacent slide-and-lock and/or passive radial elements may be integral with, e.g., cut from same piece of material, or attached by a weldless connection. In some embodiments, lengthwise adjacent radial elements may be welded together.

**FIG. 10** shows the radial element 14 before deformation, wherein the deformable region 60 exhibits a zig-zag configuration, and **FIG. 11** shows the same radial element 14 after deformation (radial expansion), wherein the deformable region 60 is stretched out to yield a linear configuration. In the illustrated embodiment of **FIGS. 10** **and** **11****,** the radial element 14 (and the stent comprising such radial element(s)) includes a deformable geometry that is incorporated into the overall stent design. The deformable regions 60 of the stent are constructed to either plastically or elastically deform during radial expansion. As the stent expands, these deformable regions can be used to achieve additional device radial expansion or increase device safety.

In one embodiment, the deformable regions plastically deform upon expansion. Advantageously, this allows for additional expansion or sizing of the stent. In another embodiment, the deformable regions elastically deform allowing for over pressurization of the stent during implantation and upon release of over pressurization, the stent returns to its intended diameter. Advantageously, this may allow for higher pressurization to treat/crack difficult lesions, while avoiding excessive vasculature injury that may occur with excessive pressure dilatations.

In yet another embodiment, the deformable regions can deform either plastically or elastically. Advantageously, this allows for an increased factor of safety as the stent reaches its maximum radial expansion limit.

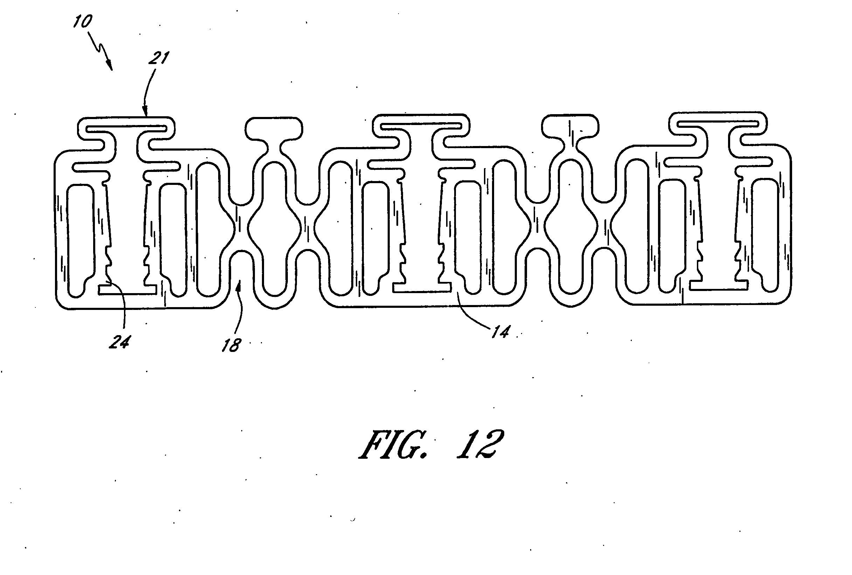

**FIG. 12** is a partial view of another embodiment of a deformable slide-and-lock stent 10. The drawing show a longitudinal module 12 with slide-and-lock 14 and passive 18 radial elements. Lengthwise adjacent radial elements within the module 12 are preferably attached by a weldless connection, and more preferably formed form the same piece of material. This embodiment comprises a deflectable tab 21, which is configured to allow it to deflect inwardly as it passes by the stops 24 in the slot 23 of a radially adjacent slide-and-lock radial element 14, but plastically returns to its prior form and alignment to prevent recoil.

### Two Sided Lockout Features

Some embodiments utilize a two-sided lockout feature, that is, stops or teeth 24 on both sides of a slot 22 (see, for example, **FIGS. 1-6**). The two-sided mechanism can be employed to increase device alignment and additionally limit off center device travel. In modified embodiments, a single-sided lockout mechanism may be utilized, that is, teeth 24 on only one side of the slot 22, (see, for example, **FIG. 7**) as needed or desired. In another modified embodiment, a two-sided mechanism can be employed by placing stops or teeth 24 on both sides of a rib element (see, for example, **FIGS. 8-9**). In this embodiment, more than one safety catch elements 54 may be employed to interact with both sides of the rib element.

### Elements to Enhance Flexibility of Stent Device

Embodiments of the slide-and-lock device design can incorporate elements which enable device flexibility in both the collapsed and expanded states. This is accomplished by providing flexible or spring elements that, for example, vary the geometry and location of the attachments between adjacent radial elements.

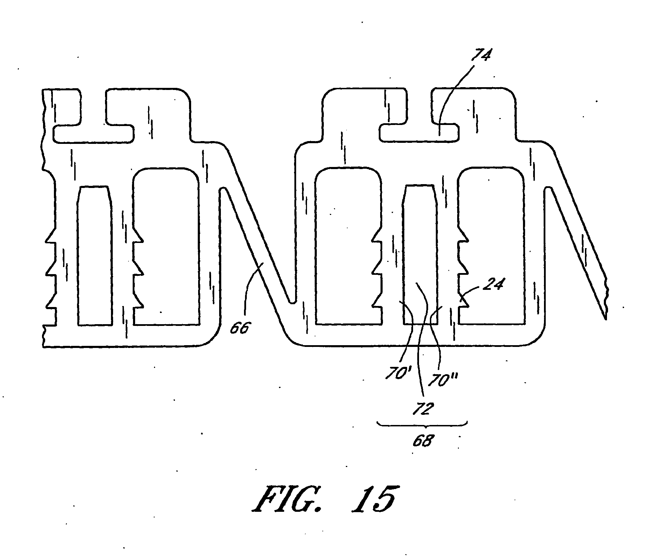

**FIG. 4****,** for example, shows flexible or spring elements 34' and 34" in the configuration of inverted diagonally aligned U-shaped members. With reference to **FIGS. 13-15****,** various alternative embodiments of flexible elements are illustrated. **FIG. 13** shows inverted U-shaped members 34' and 34" similar to those illustrated in **FIGS. 1-3****;** however, the passive radial elements in **FIG. 13** further comprise safety tabs 38 and non-mechanism slots 40. As detailed above with reference to **FIG. 4****,** the inclusion of safety tabs 38 and slots 40 without locking elements (teeth, stops or catches) may provide an additional safety against over-expansion, may help to maintain slideable engagement of radially adjacent modules, and may also maintain controlled radial expansion within the radial axis. **FIG. 14** shows a series of serpentine flexible elements 62 aligned in the longitudinal axis, contiguous with and abutting a slide-and-lock mechanism (with tab 20 and slot 22) on the proximal side and a circumferential band 64 on the distal side. The serpentine elements 62 may provide regions of enhanced flexibility between active slide-and-lock radial elements. **FIG. 15** shows linear flexible elements 66 disposed diagonally (e.g., at an angle between the radial and longitudinal axes) between slide-and-lock radial elements. Here, the slide-and-lock radial elements illustrate a variation to the tab and slot design shown for example in **FIGS. 1-3****.** The central rail 68 in **FIG. 15** comprises proximal 70' and distal 70" rail members, each with outward facing teeth 24, and an open slot 72 disposed between the rail members. The central rail 68 is adapted to slidably engage the receiving slot 74 of a radially adjacent slide-and-lock radial element, and deflect or bow inward into the open slot 72 as the receiving slot 74 ratchets past the teeth 24.

### Frangible Deployment Control Mechanism

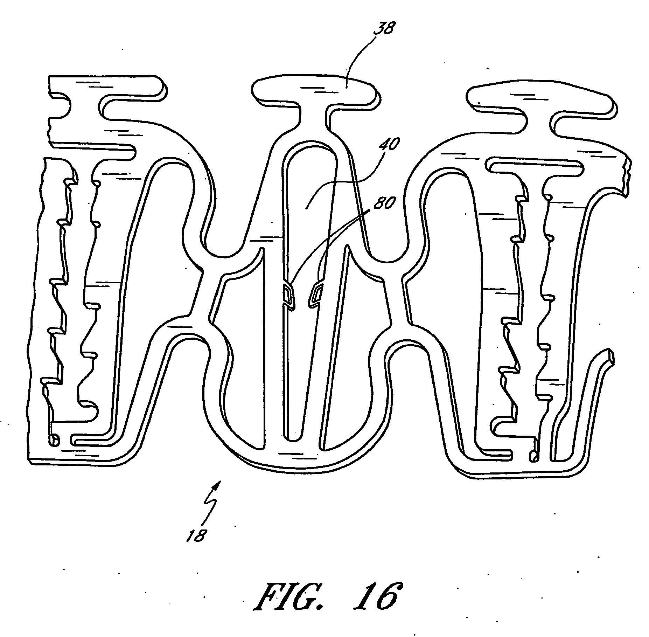

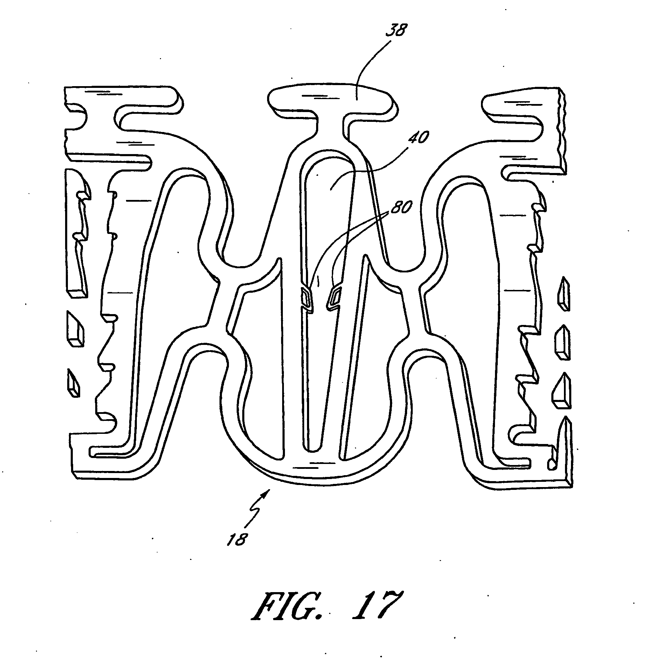

**FIGS. 16** **and** **17** are partial views of a slide-and-lock stent module incorporating a frangible deployment control mechanism. The drawings show partial modules with passive radial element 18 having a safety tab 38 and a non-locking (toothless) slot 40, similar to those shown with reference to **FIG. 4****;** however, the passive radial element in **FIGS. 16** **and** **17** include frangible members 80 that extend into the slot 40. Lengthwise adjacent radial elements are preferably attached by a weldless connection, and more preferably formed from the same piece of material. **FIG. 16** shows the passive radial element 18 before plastic deformation of the frangible members 80 and **FIG. 17** shows the passive radial element 18 after plastic deformation of the frangible members 80.

In the embodiment illustrated in **FIGS. 16** **and** **17****,** the frangible (plastically deforming) members 80 serve as a deployment control mechanism. The frangible members 80 act as a positive stop for a radially adjacent radial element having a safety tab slidably engaged within slot 40. During radial expansion, the slideably engaged radial element plastically deforms the obstructing frangible members 80 out of the path of its safety tab. This feature provides a temporary stop which allows other elements to fully expand before the temporary stop is overcome by additional radial expansion force. This feature is advantageous in facilitating uniform deployment.

### Tapered/Non-Uniform Geometries to Improve Profile and Flow

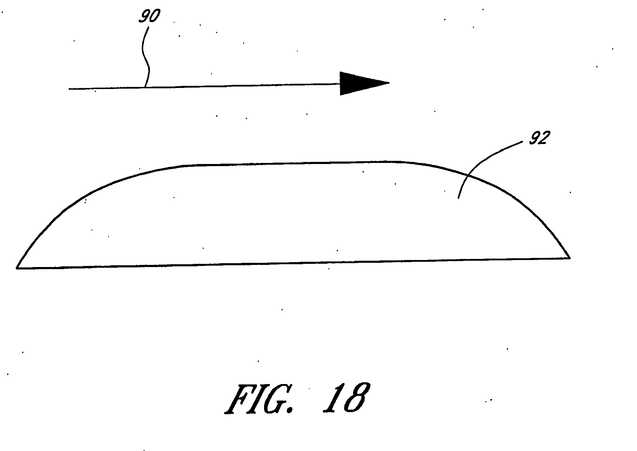

The embodiments illustrated in **FIGS. 18-21** utilize cross-sectional geometrical shapes of the radial elements that are modified/optimized to reduce the turbulence of the blood in lumen flow and/or create generally desirable blood flow characteristics. Stated differently, fluid flow principals are utilized to provide a strut or wall cross-section that is conducive to creating generally laminar and/or uniform flow characteristics.

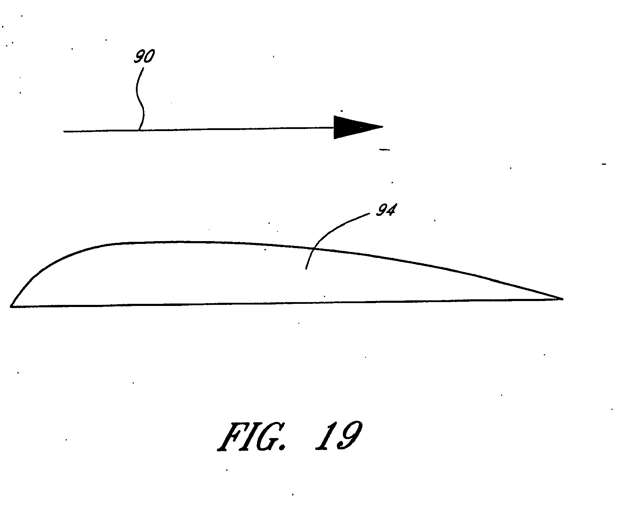

**FIG. 18** illustrates one embodiment of a streamlined strut configuration 92 for creating generally laminar and/or uniform flow characteristics where the blood flow is generally in the direction indicated by arrow 90. **FIG. 19** illustrates another embodiment of a streamlined strut configuration 94 for creating generally laminar and/or uniform flow characteristics. The direction of blood flow is generally indicated by arrow 90.

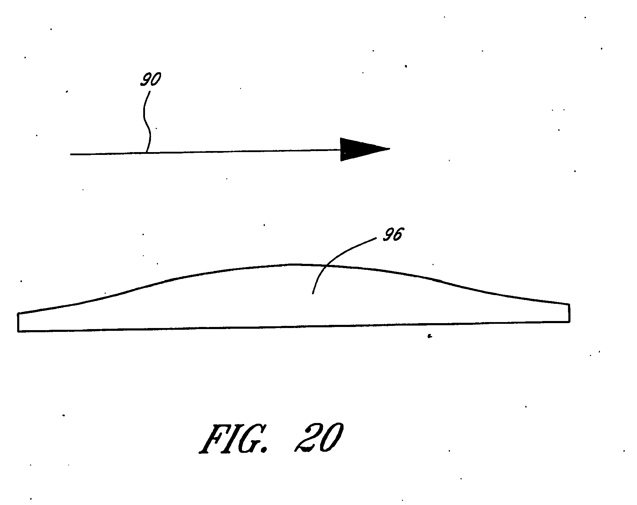

**FIG. 20** illustrates an embodiment of a strut configuration 96 that utilizes a differential thickness beam, in either the axial or circumferential direction. A differential geometry can be employed to provide thickness and strength where needed or desired and allow for increased flexibility and minimized step down as needed or desired. The direction of blood flow is generally indicated by arrow 90.

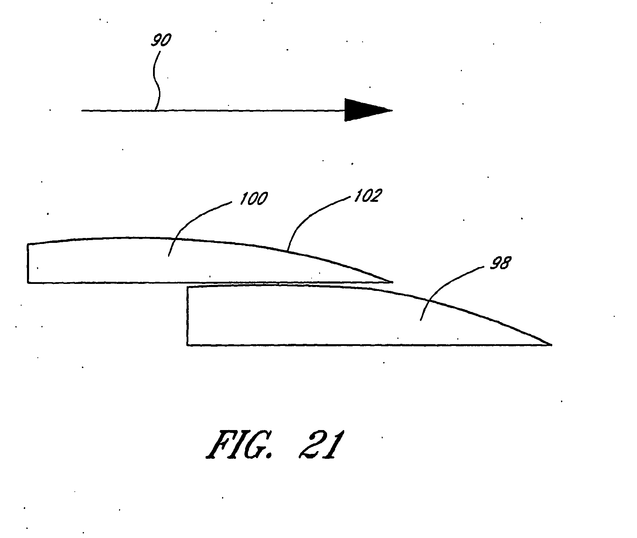

**FIG. 21** illustrates an embodiment that utilizes the streamlining concept to reduce step down effects between overlapping elements, such as elements 100 and 98. A tapered edge 102 allows the element 100 to blend into the underlying element 98 thereby, and advantageously, creating a substantially non-stepped transition point and eliminating any large step differential between the elements 100 and 98.

In another advantageous feature, it will be appreciated that preferred embodiments provide very efficient surface coverage, which is particularly advantageous when the stent is used with a therapeutic agent. More particularly, the slide-and-lock mechanism is configured such that virtually all the surface area of the locking elements is in contact with the inner wall of the body lumen. Accordingly, the preferred embodiments allow for greater surface coverage as compared with existing stent configurations. When compared with other stent configurations, such as those utilizing deformable struts, the surface coverage may be increased to as much as 25% to 70% without compromising stent performance or flexibility. Because the stent shape of various preferred embodiments provides excellent surface coverage, a larger amount of the therapeutic agent may be delivered to the surrounding tissue. As a result, the agent may be used more effectively, thereby increasing the therapeutic effect. Alternatively, the therapeutic agent may be used in a lower concentration, thereby reducing local toxicity.

### Solid Wall Stents and Grafts

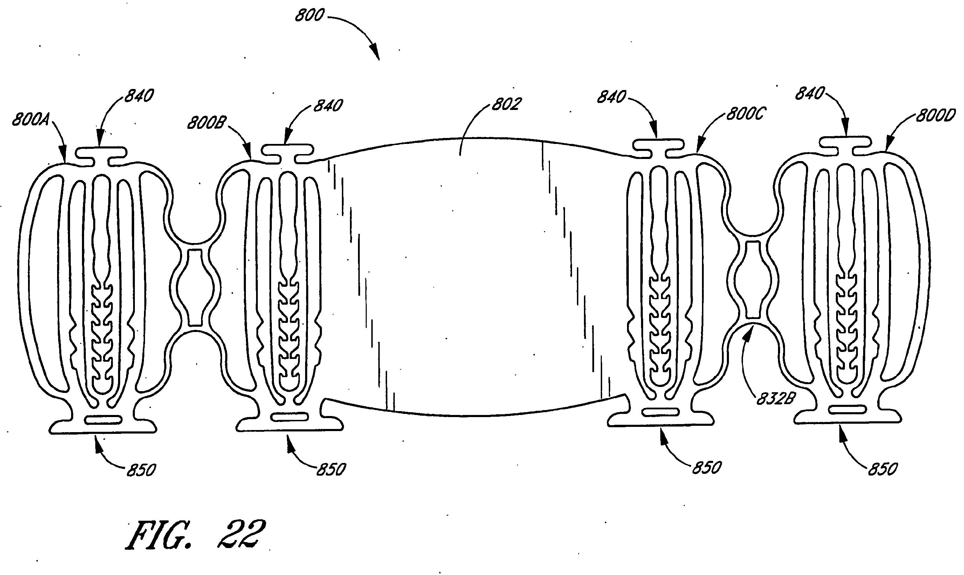

With reference now to **FIG. 22****,** another module or row 800 of radial elements 800A-800D is illustrated that may be used alone or in combination with similar elements to provide an expandable stent structure. In many respects, the module or row 800 of radial elements 800A-800D is similar to the modules described above (See e.g., FIGS. 1-3). However, in this embodiment, the flexible body is formed with a solid wall 802 along a central portion of the row for providing enhanced surface coverage in a desired region of a body lumen. In variations to the embodiment illustrated in **FIG. 22****,** the solid wall 802 may be disposed anywhere along the longitudinal length of the module or row. Moreover, in some embodiments it may be preferred to employ more than one solid wall along the length of the module. These solid wall regions may be adjacent to one another or separated.

More particularly, the solid wall 802 is preferably fabricated from an impermeable material and is configured to provide substantially complete coverage along a portion of a body lumen. In preferred embodiments, the solid wall 802 extends along the longitudinal axis at least 2 millimeters. Accordingly, this embodiment is particularly well suited for placement along a vascular anomaly, such as a vascular aneurysm, for supporting or sealing off a particular region along a vessel.

In the illustrated embodiment, each radial element 800A-800D comprises a locking tab 812 that interacts with teeth along deflectable rails for providing a locking mechanism. In some embodiments, e.g., where the stent is fabricated from a shape-memory material (e.g., Nitinol), each radial element 800A-800D may include a hold-down tab 850 sized to be releasably held within a recess for providing a hold down mechanism. It should be appreciated that a wide variety of locking mechanisms and hold-down mechanisms may be used (See e.g., those detailed in co-pending US Application No. 10/897,235), and that the illustrated embodiment is merely for the purpose of description. Flexible coupling members 832A, 832B may be provided between individual elements to provide enhanced flexibility. In one preferred embodiment, the module or row 800 of elements is fabricated from a shape memory material to provide crush-recoverability. During use, the radial elements comprising the module or row 800 are preferably slidably interconnected with other similar radial elements in circumferentially adjactent modules or rows to provide a balloon expandable stent However, in an alternative configuration, the element 800 of **FIG. 22** may be wrapped onto itself to provide an expandable stent.

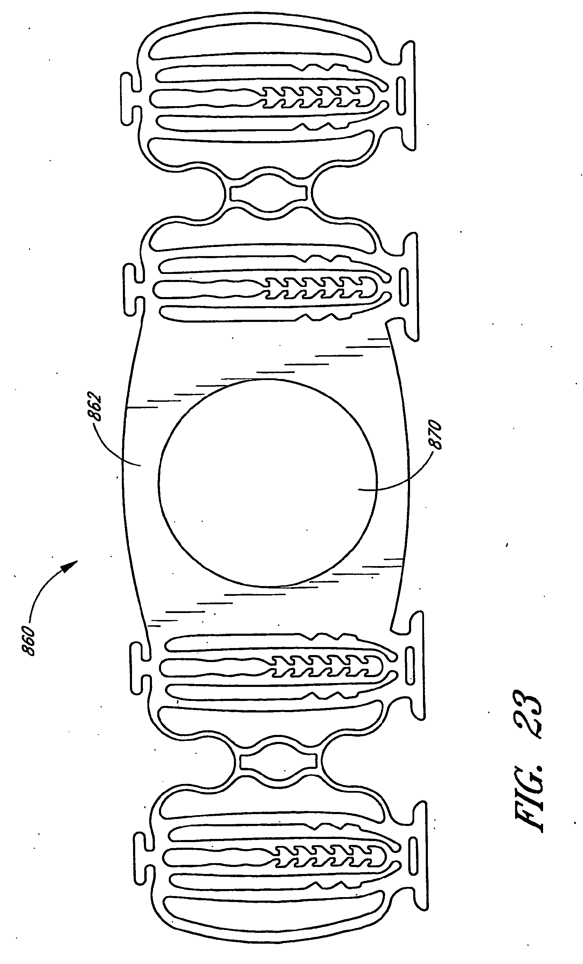

With reference now to **FIG. 23****,** an alternative row 860 is illustrated which further comprises an opening 870 (e.g., a circular hole) formed in the wall portion 862. The opening is preferably provided for allowing fluid communication through the wall 862. Accordingly, this variation 860 is particularly well suited for treating a lesion along a vessel bifurcation. The row 860 may be interconnected with one or more rows 800 of the type described above with respect to **FIG. 22** to provide an expandable stent having a solid central portion formed with an opening. When deployed, the stent may be advantageously used to ensure the patency of a main vessel while allowing blood to flow into or out of a branch vessel. In yet other variations, the wall may be permeable or a filter may be provided along the opening 870 for preventing emboli or other debris from passing through the opening.

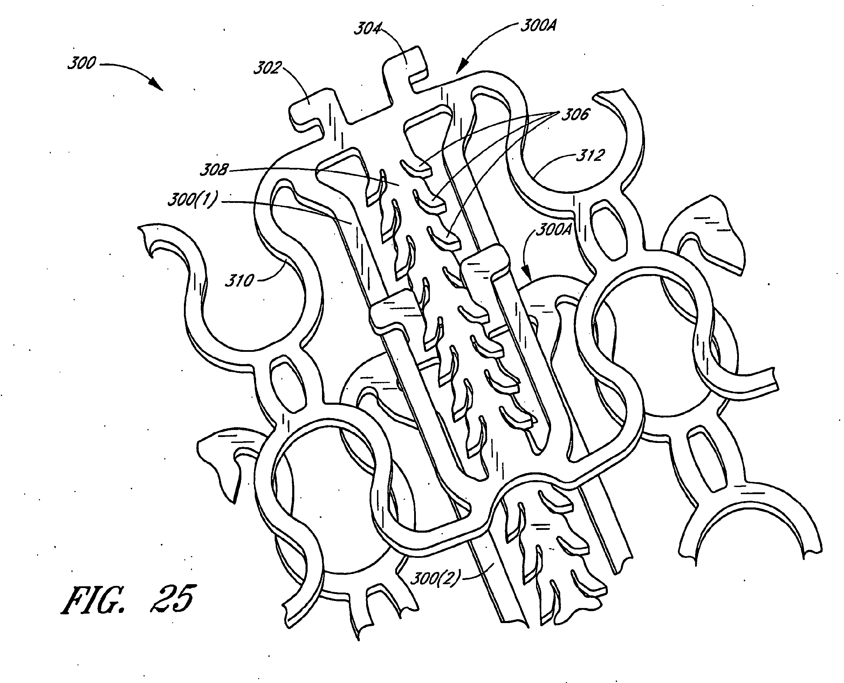

Of course, it will be appreciated that deflectable teeth may be used in those embodiments shown in **FIGS. 22-23****,** rather than deflectable rails or members (as shown), to provide the stent with mono-directional expansion. A detailed description of deflectable teeth and accompanying illustrations are provided below with reference to **FIGS. 25-27****.**

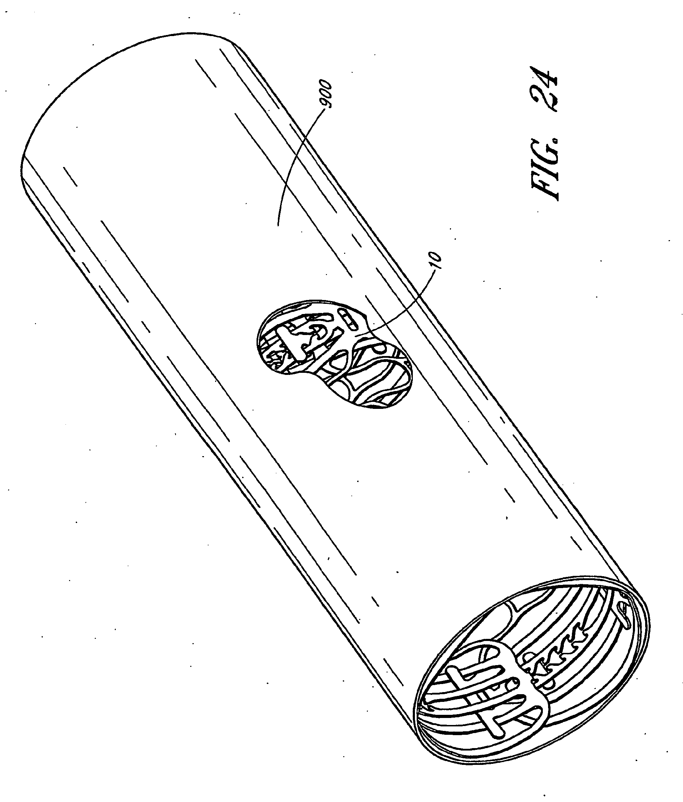

In yet another variation, stent embodiments may also be useful in vessel grafts, wherein the stent is covered with a sheath formed at least in part from either a polymeric material, such as expanded PTFE, or a natural material, such as fibrin. One variation of a graft
is illustrated in **FIG. 24****.** The tubular graft comprises an expandable stent 10 of the type described herein with reference to **FIGS. 1-23** and 25-35 and a polymeric sheath 900. Because of the low profile, small collapsed diameter and great flexibility, stents made in accordance with this embodiment may be able to navigate small or torturous paths. Thus, this variation may be useful in coronary arteries, carotid arteries, vascular aneurysms (when covered with a sheath), renal arteries, peripheral (iliac, femoral, popliteal, subclavian) arteries. Other nonvascular applications include gastrointestinal, duodenum, biliary ducts, esophagus, urethra, tracheal and bronchial ducts.

### Deflectable Teeth Lockout Mechanisms

In yet another alternative embodiment, it will be appreciated that deflectable teeth may be used, rather than deflectable rails or members, to provide the locking mechanism that facilitates mono-directional expansion. For example, **FIG. 25** illustrates a portion of another stent embodiment 300 wherein two radial elements 300(1), 300(2) are slidably interconnected. Each radial element is provided with a rail 308 having a plurality of deflectable teeth 306. Similar radial elements may be coupled via flexible linkage elements 310, 312 to provide a stent having a desired axial length. In this embodiment, the engagement means comprise locking tabs 302, 304 which are configured to slide circumferentially within elongate slots surrounding the rail, and to slide along the sides of the deflectable teeth 306. Each of the teeth is sufficiently flexible such that the teeth may deform inward toward the rail 308 (i.e., within the plane of the radial element) for allowing the locking tabs 302, 304 to pass in one direction. However, due to the angle of the teeth, the locking tabs are prevented from moving in the other direction, thereby providing yet another preferred mechanism for maintaining the stent in the expanded condition after deployment.

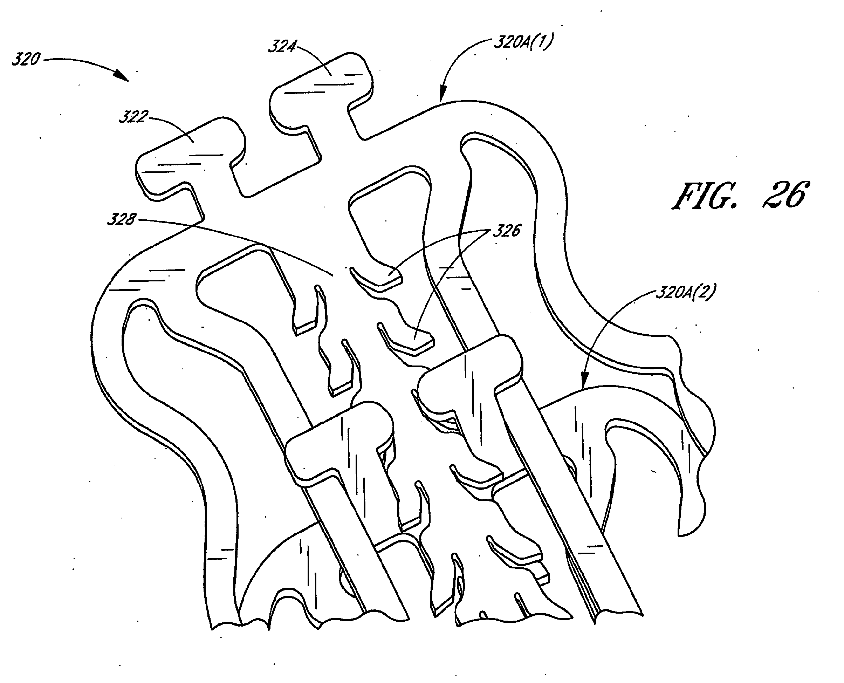

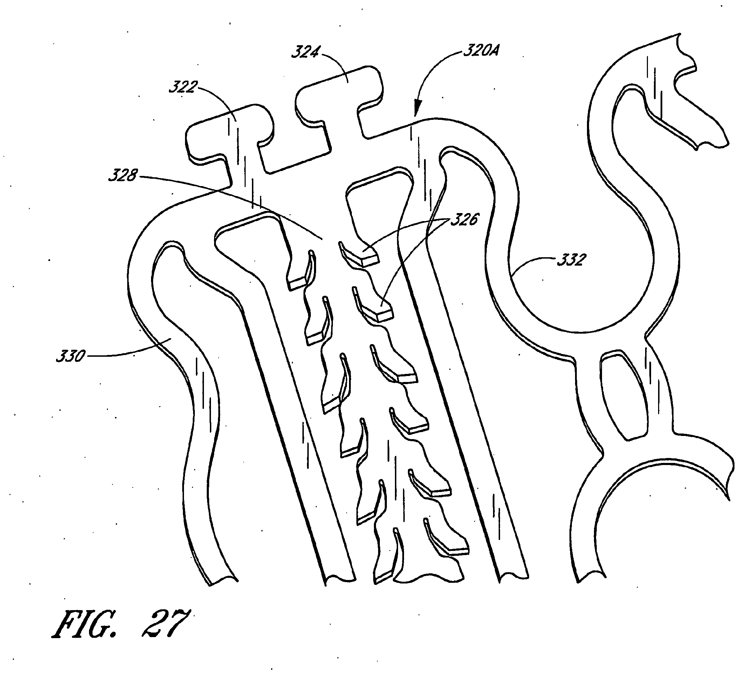

With reference now to **FIG. 26****,** a portion of another preferred stent embodiment 320 is illustrated wherein radial elements 320(1), 320(2) are slidably interconnected. Similar to the embodiment just described, each radial element is provided with a rail 328 having a plurality of deflectable teeth 326. However, in this embodiment, each of the teeth is angled upward and is configured to deflect downward (i.e., in a radial direction), rather than inward toward the rail as discussed with respect to FIG. 25. As the locking tabs 322, 324 slide along the deflectable teeth 326, the teeth are caused to deflect downward for allowing the tabs 322, 324 to pass over the teeth 326 during deployment. However, due to the angle of the teeth, the locking tabs may only move in one direction. More particularly, if a compressive force pushes the radial elements 320(1), 320(2) back toward the collapsed condition, the locking tabs 322, 324 will abut against the teeth 326, thereby preventing further relative movement. For additional reference, FIG. 27 illustrates radial element 320(1) in isolation. Flexible linkage elements 330, 332 allow multiple radial elements to be joined to form a row.

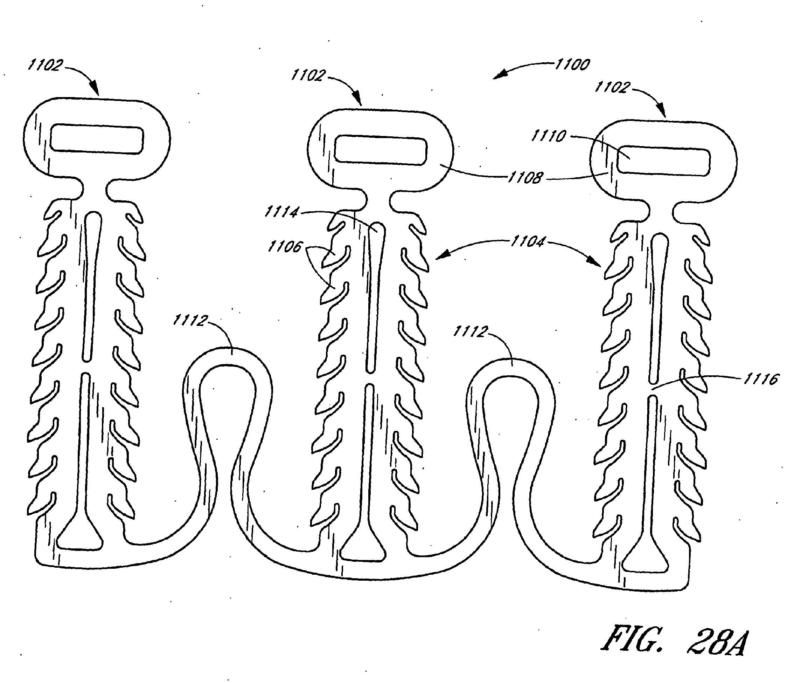

With reference to **FIGS. 28A-C,** another embodiment of the slide-and-lock stent is illustrated, wherein the locking mechanism comprises deflectable teeth disposed on rails similar to those shown in **FIGS. 25-27****;** however, after assembly, a rail is slidably engaged within engagement means comprising a closed loop which defines a slot. FIG. 28A shows a plan view of such a module 1100 comprising three radial elements 1102. Each radial element comprises a rail 1104 comprising a plurality of deflectable teeth 1106, and a closed loop 1108, which forms a slot 1110 configured to slidably engage a rail 1104 from a circumferentially adjacent radial element 1102 in a circumferentially adjacent module or row 1100. The slot 1110 is also configured to accept the loop 1108 and rail 1104 from the adjacent radial element during assembly, such that circumferentially adjacent modules can be slidably interlocked without welding or bonding of any sort required. In the illustrated embodiment, the longitudinally adjacent radial elements 1102 are connected to one another via flexible linkage elements 1112. Of course, any linkage configuration may be substituted without departing from the inventive elements of this embodiment. The illustrated rails 1104 have a central gap 1114 that may be configured to provide more or less deflection of the teeth 1106 as they pass through the slot 1110 during expansion. One or more bridges 1116 may connect the two sides of the divided rail. Generally, the more bridges the less deflection the rail may offer.

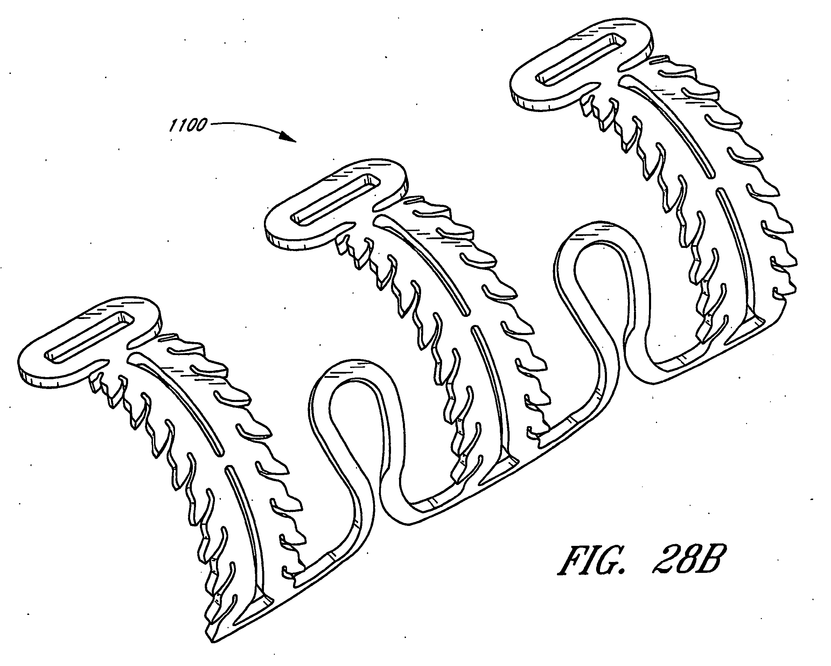

**FIG. 28B** shows the same module 1100 illustrated in **FIG. 28A****,** except it has been bowed to show how the module comprises a portion of the circumference of a stent assembled from 2 or more such modules.

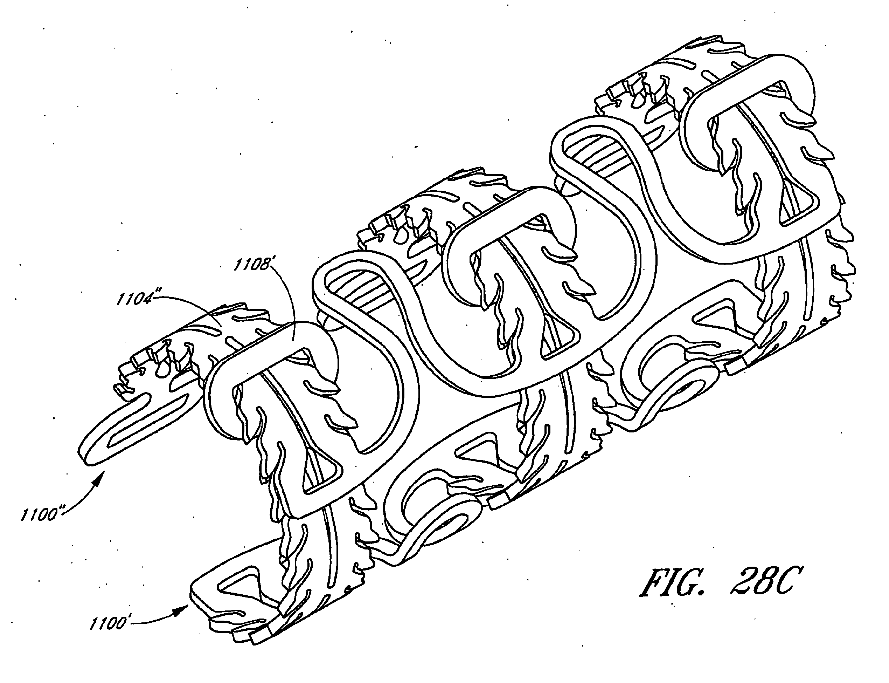

**FIG. 28C** illustrates a partial stent comprising two modules 1100' and 1100" like those showin in **FIGS. 28A** **and** **28B****.** It will be appreciated that the rail 1104" from module 1100" is slidably engaged in the slot 1110' formed in closed loop 1108' from module 1100'.

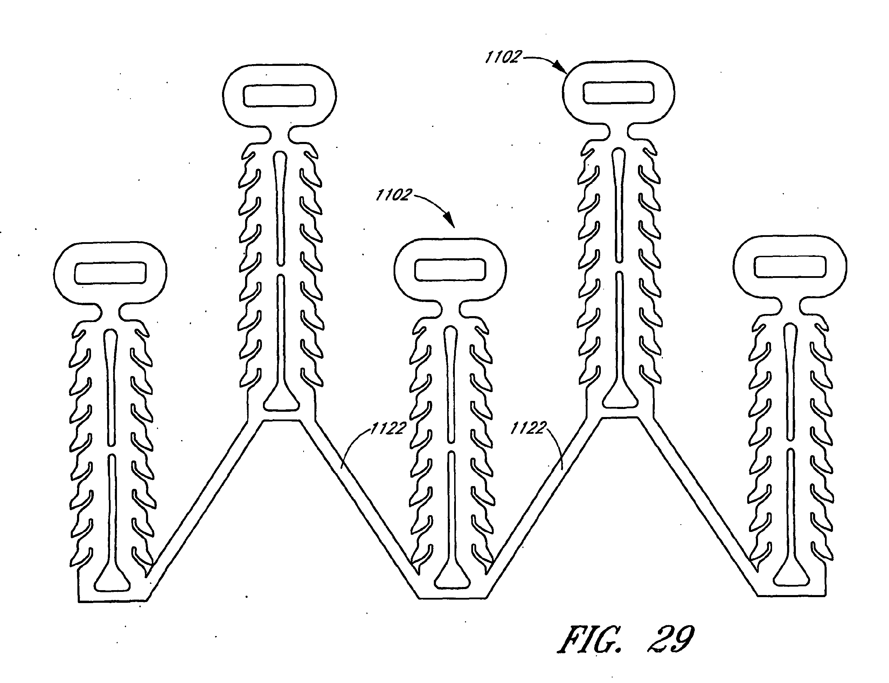

A variation to the deflectable tooth slide-and-lock module shown in FIGS. 28A-C is illustrated in **FIG. 29****,** wherein the longitudinally adjacent radial elements 1102 are circumferentially offset by angled linkage elements 1122.

### Deflectable Teeth Lockout Mechanisms Having Tapered Thicknesses

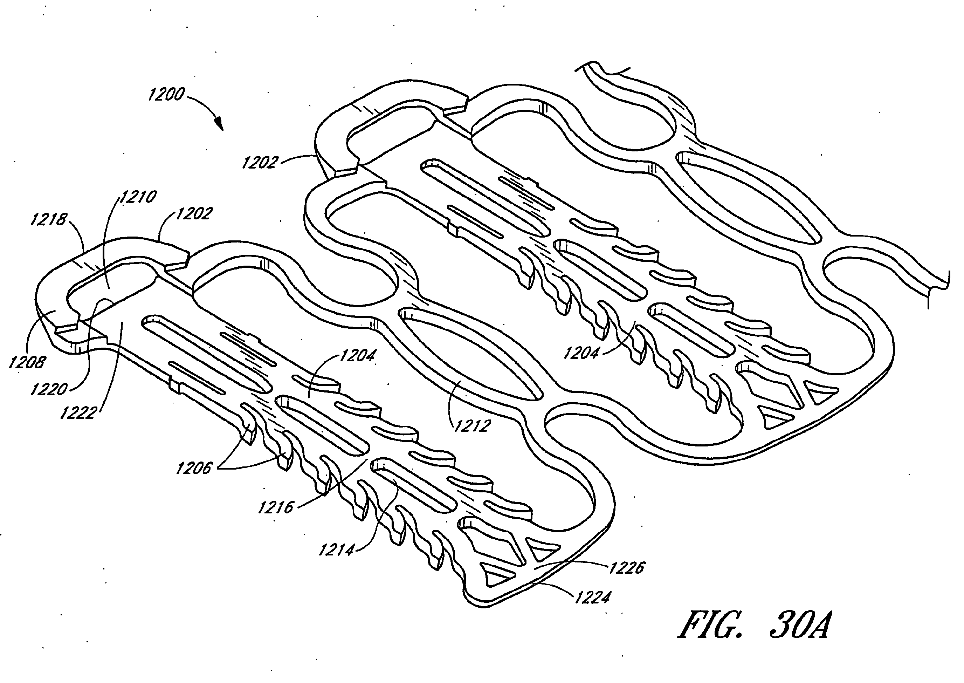

Referring now to **FIGS. 30A-C,** another embodiment of the slide-and-lock stent is illustrated. The innovative embodiments illustrated in **FIGS. 30A-C** are similar to **FIGS. 25-29****,** wherein the locking mechanism comprises deflectable teeth disposed on rails, and particularly similar to **FIGS. 28A-29****,** wherein after assembly, a rail is slidably engaged within engagement means comprising a closed loop which defines a slot However, the embodiments shown in **FIGS. 30A-C** further provide that the radial elements of the stent are uniquely configured to include tapered sections in the circumferential direction in order to reduce the radial thickness of the stent at the overlapping sections of circumferentially adjacent modules. Such an embodiment may also provide a reduced cross-sectional profile compared to other stents that do not have radial elements with such tapered sections. Furthermore, it is also contemplated that the radial elements may be configured to provide an approximately constant radial thickness of the stent despite overlap of circumferentially adjacent radial elements.

**FIG. 30A** shows a perspective view of such a module 1200 comprising two radial elements 1202, as similarly illustrated in **FIG. 28A****.** Each radial element 1202 comprises a rail 1204 comprising a plurality of deflectable teeth 1206, and a closed loop 1208, which forms a slot 1210 configured to slidably engage a rail 1204 from a circumferentially adjacent radial element 1202 in a circumferentially adjacent module or row 1200. The slot 1210 is also configured to accept the loop 1208 and rail 1204 from the adjacent radial element during assembly, such that circumferentially adjacent modules can be slidably interlocked. In such an embodiment, welding or bonding of may be required to properly adjoin adjacent modules. In the illustrated embodiment, the longitudinally adjacent radial elements 1202 are connected to one another via flexible linkage elements 1212. The flexible linkage elements 1212 may be similarly configured to other as described herein. Of course, various linkage configurations may be substituted without departing from the inventive elements of this embodiment. The illustrated rails 1204 may have a central gap 1214 that may be configured to provide more or less deflection of the teeth 1206 as they pass through the slot 1210 during expansion. One or more bridges 1216 may connect the two sides of the divided rail. Generally, the more bridges the less deflection the rail may offer.

According to the unique aspect of the invention mentioned above and as illustrated in the embodiment of **FIGS. 30A-C,** a plurality of modules may be uniquely configured to include radial elements with tapered sections in order to reduce the radial thickness of the stent at the overlapping sections of circumferentially adjacent radial elements. Stents with non-tapered radial elements have a "double radial thickness" where the modules circumferentially interlock and overlap. According to an embodiment of the present invention, in order to overcome the "double radial thickness" deficiency, each radial element may be formed to include tapered sections that nest within respective tapered sections to reduce the cross-sectional profile of the stent.

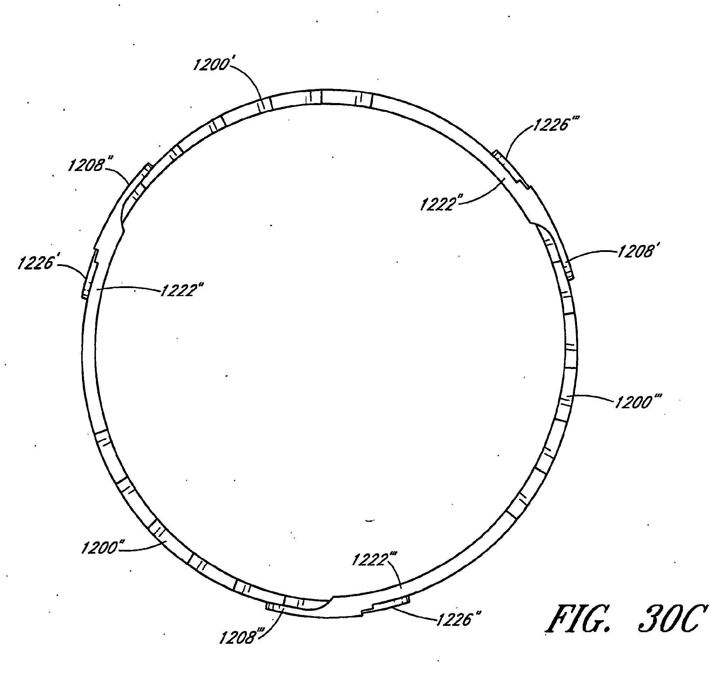

For example, as illustrated in **FIG. 30A****,** the thickness of the loop 1208 of the radial element 1202 may taper in the circumferential direction approaching a distal edge 1218 of the loop 1208. Similarly, the thickness of the rail 1204 of the radial element 1202 may also taper in the circumferential direction approaching a distal edge 1220 of an upper portion 1222 of the rail 1204. Finally, the thickness of the rail 1204 of the radial element 1202 may also taper in the circumferential direction approaching a distal edge 1224 of a lower portion 1226 of the rail 1204. Therefore, when the stent is expanded, as illustrated in **FIG. 30C****,** these tapered sections of the loop 1208 and the upper and lower portions 1222, 1226 of the rail 1204 may nest with respective tapered sections to reduce the cross-sectional profile of the stent.

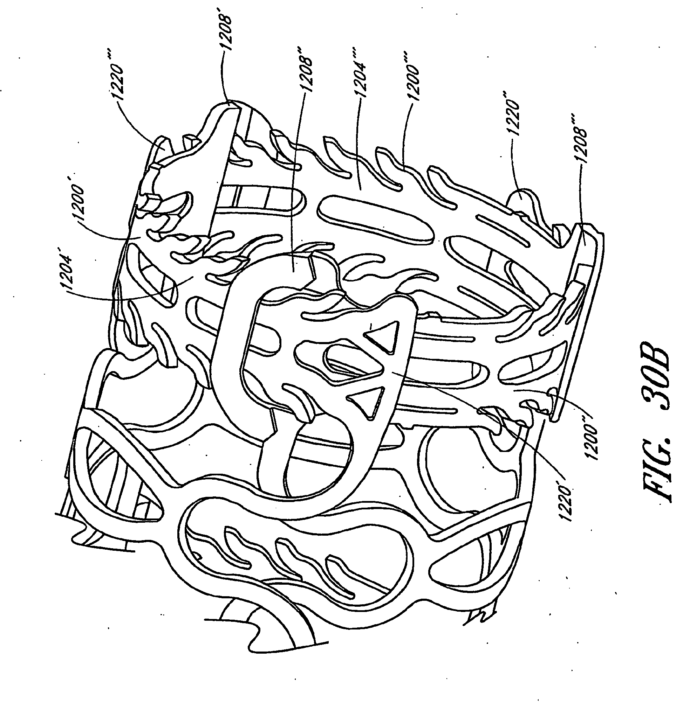

In an illustration of the nesting of the tapered sections, shown in **FIGS. 30B-C,** the stent may comprise modules 1200', 1200", and 1200'" which combine to form the tubular member of the stent. In this embodiment, the loop 1208" of the radial element 1200" engages the respective rail 1204' from the adjacent radial element 1200' to facilitate slidable interlocking of adjacent modules 1200" and 1200'. As shown in **FIG. 30C****,** when the stent is expanded, the tapered section of the loop 1208" and the tapered section of the upper portion 1222" of the module 1200" nest with the tapered section of the lower portion 1226' of the module 1200'.

Referring still to **FIG. 30C****,** the radial thickness of the expanded stent may be a maximum of approximately 1½ of the thickness of the non-tapered portion of the rail 1204. For example, as shown in **FIGS. 30A** and 30C, the maximum thickness of the loop 1208 may be approximately 1½ of the thickness of the non-tapered portion of the rail 1204. As shown, the loop 1208 then tapers from this maximum thickness toward a thickness of ½ of the thickness of the non-tapered portion of the rail 1204 in the circumferential direction approaching the distal edge 1218 of the loop 1208. However, the maximum combined radial thickness is preferably also approximately 1½ of the thickness of the non-tapered portion of the rail 1204. The combined radial thickness may be measured such as at the distal edge 1218 of the loop 1208 and at the distal edge 1220 of the rail 1204. Thus, at the distal edge 1218 of the loop 1208, where the loop 1208 nests with the non-tapered portion of the rail 1204, the combined thickness of the loop 1208 and the rail 1204 will be 1½ of the thickness of the non-tapered portion of the rail 1204 (the loop 1208 being ½ of the thickness of the rail 1204 at this intersection). In summary, the thickness of the radial element 1202 along the non-tapered portion of the rail 1204 may be a first thickness, and the tapered sections of the module 1200 may taper toward approximately a second thickness. The second thickness is preferably approximately one-half of the first thickness. For example, the first thickness (the thickness of the non-tapered portion) may be approximately 0.1016 mm (0.0040) inches, and the second thickness may be approximately 0.0508 mm (0.0020) inches. Accordingly, the maximum combined radial thickness of the first and second thicknesses in the overlapping sections of the stent preferably does not exceed 1½ of the thickness of the non-tapered portion of the rail 1204. However, it is contemplated that the first thickness may vary by as much as 0.0254 mm (0.0010)inches, or that the second thickness may vary by as much as 0.0127 mm (0.0005) inches. In this regard, the second thickness may sometimes be greater than ½ of the first thickness, thus resulting in a combined thickness of slightly greater than 1½ of the first thickness. Other variations due to manufacturing variations may result; however, the general teachings herein may nevertheless be utilized to provide for a stent with a reduced cross-sectional profile relative to stents without tapered radial elements.

Therefore, the individual thicknesses of the respective ones of the rail 1204, the loop 1208, and the upper portion 1222 of the rail 1204 may gradually taper corresponding to the thicknesses of the respective ones of the rail 1204, the loop 1208, and the upper portion 1222 of the rail 1204, with which it is nested, in order to ensure that the maximum combined thickness in the overlapping sections does not exceed 1½ of the thickness in the non-tapered portion of the rail 1204.

Such an embodiment may also provide a reduced cross-sectional profile compared to other stents that do not have modules with such tapered sections. The tapered sections provide the stent with an increased interior diameter and a decreased exterior diameter. For example, if the tapered stent includes overlapping sections that measure approximately 0.1524 mm (0.0060) inches (as opposed to 0.1032 mm (0.0080) inches on a non-tapered stent, as discussed above), then the exterior diameter of the stent may be reduced by at least 0.0508 mm (0.0020) inches at at least two or three overlapping sections (see **FIG. 30C****),** and perhaps 0.1016 mm (0.0040) inches, depending on the configuration of the deployed stent. Furthermore, the interior diameter of the stent may likewise increase due to two considerations. First as illustrated in **FIG. 30C****,** the interior diameter of the module may be increased by 0.0508 mm (0.0020) inches at at least two or three overlapping sections, and as much as 0.1016 mm (0.0040) inches relative to non-tapered stents, depending on the configuration. Second, it is contemplated that the decrease in the exterior diameter may allow the stent to be further expanded, thus further increasing the interior diameter of the tapered stent relative to a non-tapered stent As a result, the deployed tapered stent may provide better flow characteristics (such as tending to ensure laminar flow of the blood within the stent due to tapered interior geometries), as well as increased blood flow rate due to the elimination of a portion of the radial thickness of the stent. Indeed, by including the tapered sections along the module 1200, the inside diameter of the stent may be increased in the two ways mentioned above, without requiring a larger exterior diameter than the non-tapered stent. Thus, the stent may be more efficient that previous non-tapered stents.

In addition, the exterior of the stent may be smooth (i.e. lower surface roughness than stents with non-tapered modules) due to the decreased thickness of both the lower portion 1226 of the rail 1204 and the loop 1208, which are typically disposed on the exterior of the stent, as shown in **FIGS. 30B-C.** The reduced cross-sectional profile also allows the exterior of the stent to more fully contact the interior walls of the body lumen. Therefore, the insertion and/or removal of the stent may be facilitated by the inclusion of the tapered areas.

In accordance with another advantageous aspect of the present invention, the addition of the tapered sections may also provide for a reduced cross sectional profile when the stent is in its "crimped down" undeployed state. As discussed above, the exterior diameter of the deployed stent may be decreased by as much as a single thickness of the non-tapered portion of the rail 1204. However, an even more dramatic reduction is possible in the size of the undeployed stent. Through the use of tapered sections, the outside diameter of the undeployed stent may be reduced in the order of 0.1016 mm (0.0040) to 0.3048 (0.0120) inches (1 to 3 times the thickness of the non-tapered rail 1204). For example, the modules 1202 of the stent curl inwardly multiple times when in bundled (i.e. undeployed) state. In this state, the tapered sections of the module are up to 0.0508 mm (0.0020) inches thinner than a non-tapered portion. Thus, each layer of a tapered section represents a decrease of 0.0508 mm (0.0020) inches in the outside diameter of the bundled (i.e. undeployed) stent. Decreases of between 0.1016 mm (0.004) and 0.3048 mm (0.0120) inches are thus possible when compared to non-tapered stents. Thus, the tapered sections of the stent may also greatly facilitate insertion of the undeployed stent due to the reduced cross-sectional profile of the undeployed stent.

Furthermore, it is also contemplated that the modules may be configured to provide an approximately constant radial thickness of the stent despite overlap of circumferentially adjacent modules. Thus, the respective thicknesses of the respective ones of the rail 1204, the loop 1208, and the upper portion 1222 of the rail 1204 may gradually taper corresponding to the thicknesses of the respective ones of the rail 1204, the loop 1208, and the upper portion 1222 of the rail 1204, with which it is nested, in order to ensure that the maximum combined thickness in the overlapping sections does not exceed the thickness in the non-tapered portion of the rail 1204. Such a configuration may tend to improve the cross-sectional profile of the stent by maximizing the interior area of the stent while maintaining or even reducing the exterior profile (i.e. exterior area) of the stent. This improvement may thus result in improved flow rate relative to stents having non-tapered modules.

### Serrated Surface Lockout Mechanisms

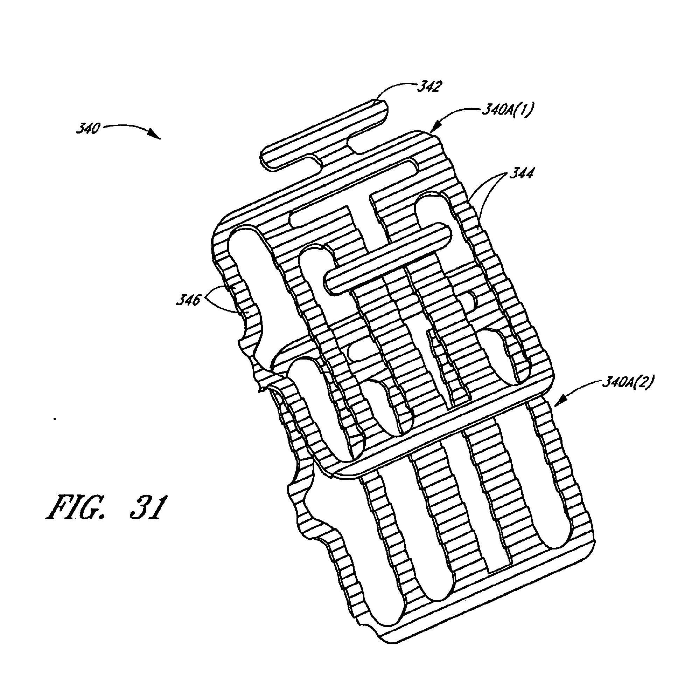

With reference now to **FIG. 31****,** a portion of another stent embodiment 340 is illustrated wherein radial elements 340(1), 340(2) are slidably interconnected. Each radial element is provided with an outer surface formed, at least in part, with a series of serrations or ridges. More particularly, the surfaces comprise a series of valleys 344 and ridges 346. In the illustrated configuration, a locking tab 342 of radial element 340(2) slides along the surface of radial element 340(1). The locking tab 342 is formed with a thin neck portion 350 and a wider head portion 352. The neck portion 350 is configured for allowing the head 352 to deflect outward in a radial direction. The shape of the valleys 344 and ridges 346 allows the head 352 of the locking tab 342 to ratchet along the surface of the adjacent element in only one direction, thereby providing a locking means to maintain the stent in the expanded condition. Although the ridges and valleys are only necessary along the region wherein the locking tab slides, each of the radial elements may be formed with a continuous contoured surface for ease in manufacturing. In one variation, the shaped bottom surface of the first element 340(1) may slide along the top surface of the shaped second element 340(2) for providing the desired ratcheting effect. In this variation, the tab 342 may be used primarily for interconnecting the elements in a slidable configuration.

### Variations in Lockout Mechanisms and Circumferentially Offset Radial Elements

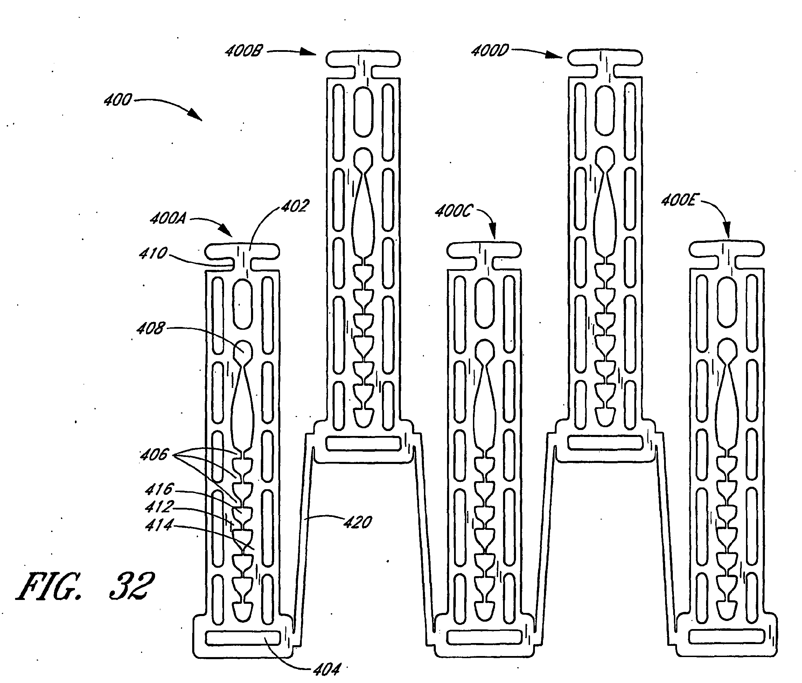

With reference now to **FIG. 32****,** another alternative module or row 400 of radial elements 400A-400E is illustrated. In this embodiment, the individual radial elements in a module or row are coupled in a staggered, circumferentially offset arrangement by a series of flexible coupling elements 420. In preferred embodiments, the illustrated module or row 400 may be slidably interconnected with other similar, circumferentially adjacent modules to provide a stent. Each of the radial elements is substantially identical and includes a locking tab 402 having a neck portion 410. Each of the radial elements further includes a containment gap 408 for holding an adjacent locking tab and a series of opposing teeth 406 along the containment gap for providing a mono-directional expansion.

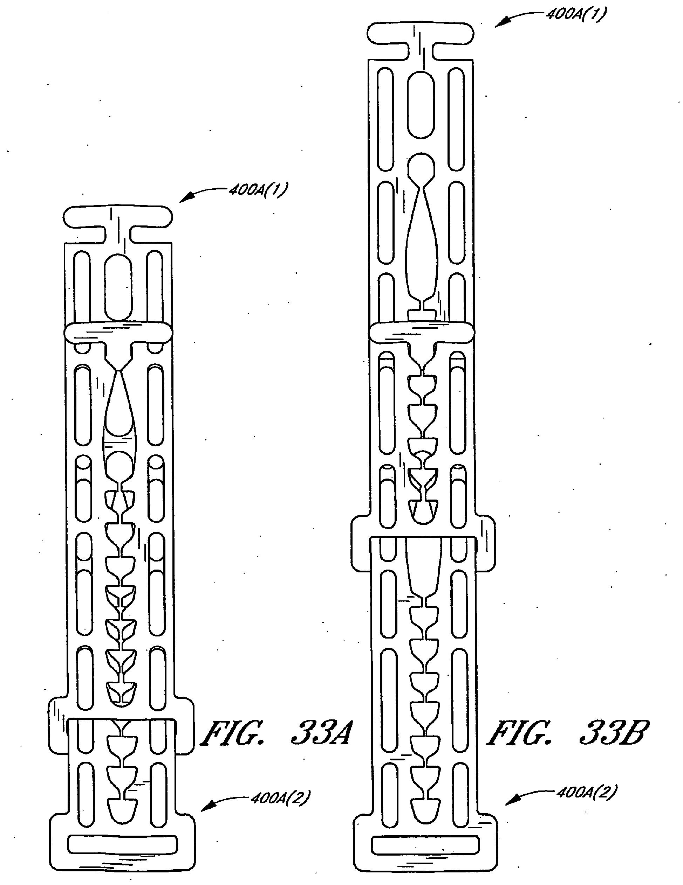

With reference now to **FIGS. 33A and 33B****,** the slide-and-lock relationship between interconnected radial elements of the type shown in **FIG. 32** is illustrated. **FIG. 33A** shows the radial elements 400A(1), 400A(2) in a collapsed configuration wherein the locking tab 402 of radial element 400A(2) is held within the containment gap 408 of radial element 400A(1). The body of radial element 400A(2) extends through a slot 404 formed in radial element 400A(1) for maintaining the elements in the desired slidable relationship. **FIG. 33B** shows the radial elements 400A(1), 400A(2) in an expanded condition. As shown in **FIG. 33B****,** the locking tab 402 of 400A(2) is disposed in the gap 416 between deflectable members 412, 414 of 400A(1) and is locked in place by teeth 406.

In one advantageous feature, a stent comprising the sliding and locking rows illustrated in **FIGS. 32** through **33B** provides improved uniformity in surface coverage due to the staggered relationship of the individual radial elements. Furthermore, the stent is capable of providing adequate support to the body lumen while minimizing the total area of surface coverage. This is a particularly advantageous feature since a large percentage of the natural inner surface of the body lumen remains exposed after stent deployment. In another advantageous feature, each radial element passes through the slot 404 of the adjacent radial element for securely maintaining the components in a slidably interlocked condition. Still further, this stent embodiment provides excellent flexibility after deployment.

As discussed above, it will be appreciated by those skilled in the art that stents constructed according to the present invention may comprise a wide variety of other slide-and-lock elements while still providing the features and advantages described herein. The slide-and-lock elements illustrated and described above are merely preferred embodiments and alternative slide-and-lock elements may be employed without departing from the scope of the invention. For example, a variety of alternative one-way locking mechanisms, which may be used to facilitate mono-directional stent expansion, can be found in Applicant's co-owned U.S. Patent Nos. 6,033,436, 6,224,626 and 6,623,521.

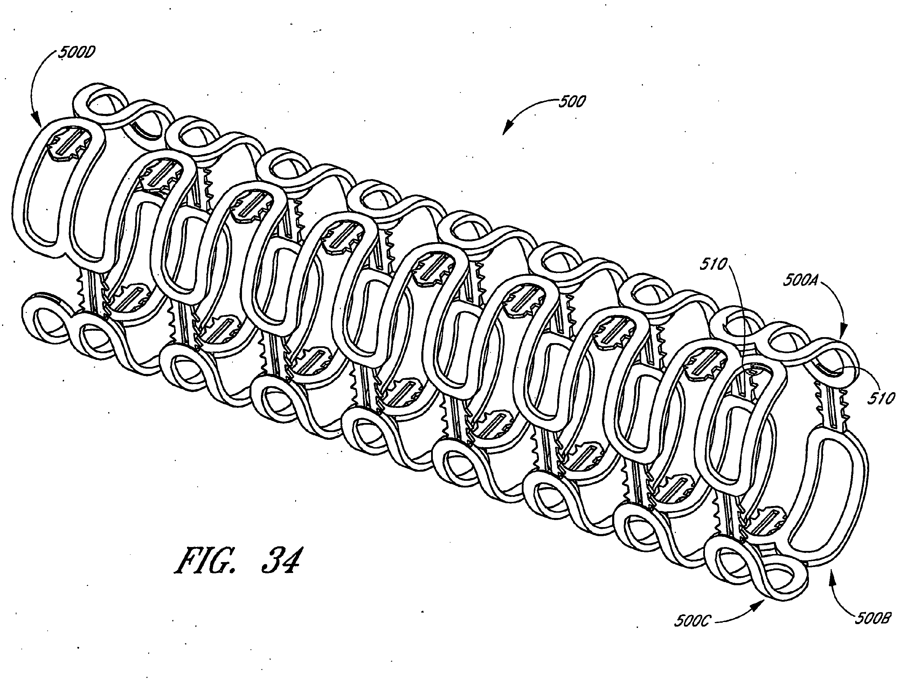

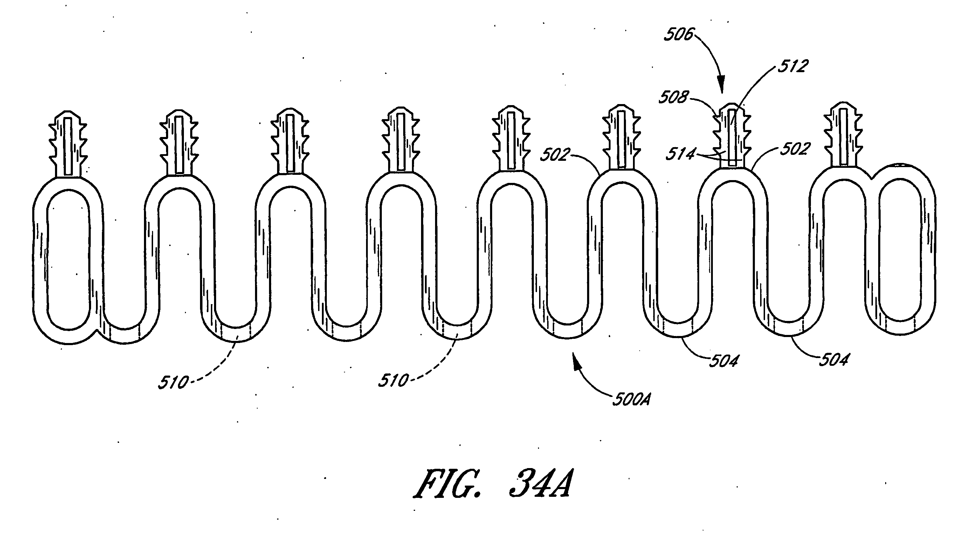

With reference now to **FIG. 34****,** yet another preferred embodiment of a stent 500 comprises alternative slide-and-lock mechanisms which are interconnected to provide a tubular member sized for deployment in a body lumen. In the illustrated embodiment, a plurality of interconnected rows 500A-500D is provided wherein each row preferably extends along the entire axial length of the stent 500. This stent configuration advantageously combines excellent longitudinal flexibility (i.e., bending) with a very high radial strength. Although the stent 500 shown in **FIG. 34** is illustrated with four interconnected rows 500A-500D, the number and length of the rows may vary to meet the particular requirements of the application.

With reference now to **FIG. 34A****,** a single row 500A comprises a structure shaped for providing the stent with excellent flexibility along the longitudinal axis. This feature allows the stent to bend during delivery and to more easily conform to the shape of a body lumen after deployment. Furthermore, this embodiment eliminates the need for flexible linkage elements. The row 500A illustrated in **FIG. 34A** includes a series of peaks 502 and valleys 504 wherein each peak is provided with a protrusion 506 and each valley is provided with a slot (e.g., see 510 **of** **FIG. 34****)** shaped for receiving an adjacent protrusion. Of course, not all peaks and valleys necessarily comprise protrusions or slots. As illustrated, each of the protrusions 506 is preferably provided with two parallel deflectable members 514 formed with a number of teeth 508. Each of the teeth 508 is formed with an angled side and a flat side. Furthermore, each of the protrusions 506 is formed with a gap 512 extending between the deflectable members 514.

When assembled, the protrusions 506 are slidably received within the slots 510 as illustrated in **FIG. 34**. The interaction between the angled teeth 508 and slots 510 is preferably configured to provide a stent 500 exhibiting mono-directional expansion. In particular, during expansion, the interaction between the teeth 508 and the slot 510 causes the deflectable members 514 to flex inward for allowing the teeth to pass through the slot 510. The deflectable members 514 are caused to flex inward because the edges of the slot act on the angled side of the teeth. However, when a force is applied in the other direction, the flat sides of the teeth abut against the edges of the slot and no inward force is produced. Accordingly, the teeth 508 are prevented from sliding back out of the slots 510, thereby maintaining the stent in the expanded condition after deployment at a treatment site.

In preferred embodiments, the force required to move the protrusions through the slots is large enough such that the stent will not inadvertently expand during delivery to the treatment site. Therefore, the stent is held down in the collapsed condition before deployment. If necessary, the assembly may be constructed such that the initial resistance produced by the first set of teeth on each protrusion is greater to ensure that the stent remains in the collapsed condition during delivery.

In an advantageous feature, each of the mating protrusions and slots may move (i.e., ratchet) independently of the others. Accordingly, in addition to providing excellent flexibility, the diameter of the stent may vary along the longitudinal axis for precisely conforming to the inner diameter of the vessel. In still another advantage, the protrusions are received within slots formed in the adjacent row. Therefore, the slide-and-lock mechanism maintains a very low profile after deployment. Indeed, in accordance with preferred embodiments, the modules comprising serpentine peaks 502 and valleys 504 may be fabricated from three or more layers of material (two outer layers and one or more inner layers), such that the slots 510 are defined by the outer layers, with a gap where at least one region of inner layers is missing, whereas the protrusions 506 are formed from the corresponding inner layer(s), with missing outer layers. Thus, as the protrusions articulate within the slots, there is no overlap of any stent elements. The thickness of the slidable articulation (between protrusions and slots) is substantially the same as the thickness of the rest of the stent.

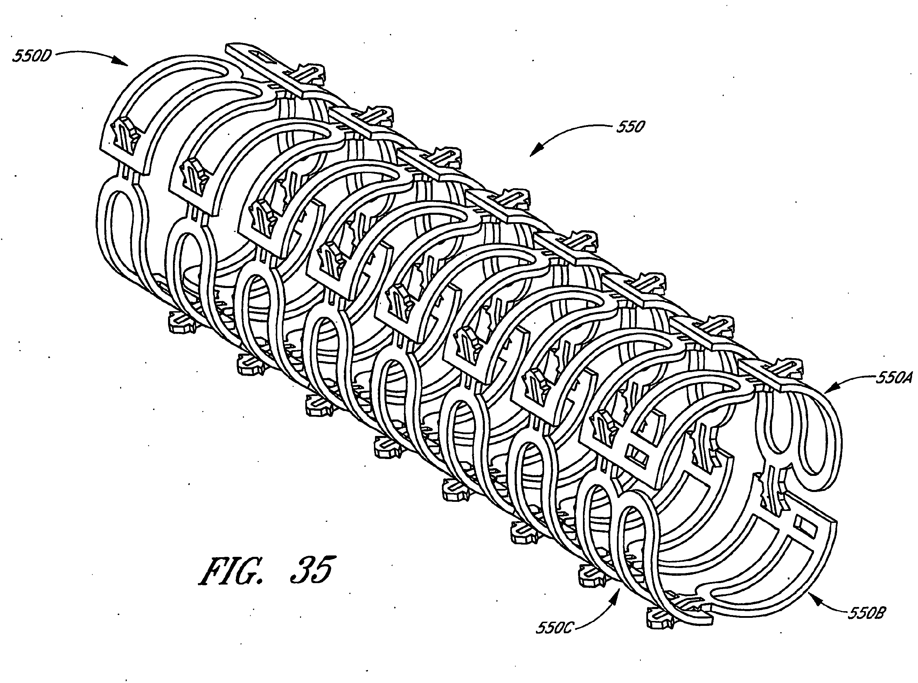

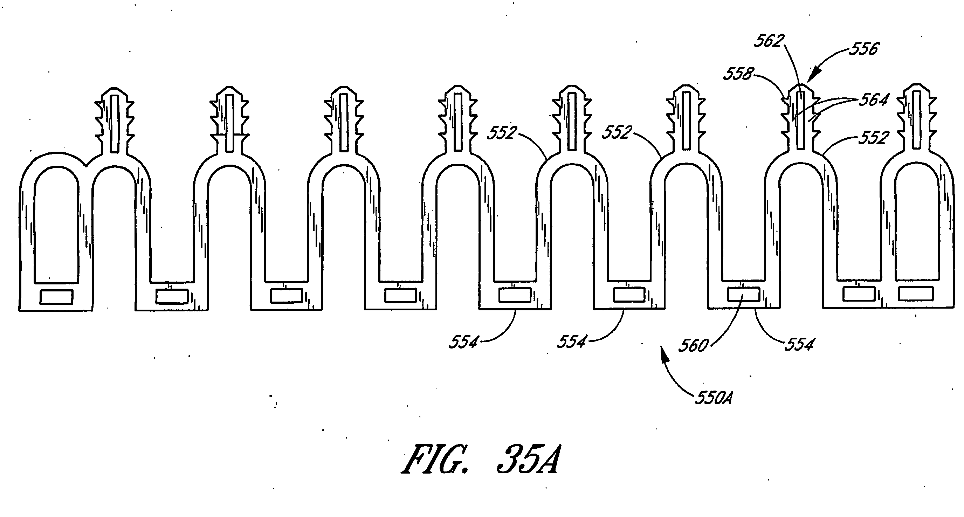

With reference now to **FIG. 35****,** stent 550 comprises yet another configuration of slide-and-lock elements which are interconnected to provide a tubular member sized for deployment in a body lumen. Similar to the stent described above with respect to **FIG. 34****,** in this embodiment, a plurality of interconnected rows 550A-550D is provided wherein each row preferably extends along the entire axial length of the stent 550. Although the stent 550 shown in **FIG. 35** is illustrated with four interconnected rows 550A-550D, the number and length of the rows may vary to meet the particular requirements of the application.

With reference now to **FIG. 35A****,** a single row 550A comprises a structure shaped for providing the stent with excellent flexibility. This feature allows the stent to bend during delivery and to more easily conform to the shape of a body lumen after deployment. The row illustrated in **FIG. 35A** includes a series of peaks 552 and valleys 554 wherein each peak is provided with a protrusion 556 and each valley is provided with a slot extending therethrough. Each of the protrusions 556 is preferably provided with two deflectable members 564 formed with a number of teeth 558. Each of the teeth 558 is formed with an angled side and a flat side. Furthermore, each of the protrusions 556 is formed with a gap 562 extending between the deflectable members 564. When assembled, the protrusions 556 are slidably received within the slots 560 as illustrated in **FIG. 35****.** The interaction between the angled teeth 558 and slots 560 is preferably configured to provide a stent 550 exhibiting mono-directional expansion. In particular, during expansion, the interaction between the teeth 558 and the slot 560 causes the deflectable members 564 to flex inward for allowing the teeth to pass through the slot 560. The deflectable members 564 are caused to flex inward because the sides of the gap act on the angled side of the teeth. However, when a force is applied in the opposite direction, the flat sides of the teeth abut against the sides of the gap and no inward force is produced. Accordingly, the teeth 558 are prevented from sliding back out of the slots 560, thereby maintaining the stent in the expanded condition after deployment at a treatment site.

With reference again to the embodiment illustrated in **FIG. 35****,** the protrusions preferably pass in a radial direction through the gaps in the adjacent rows. After deployment, an end portion of each protrusion may protrude radially outward from the tubular member, as shown in **FIG. 35****.** The end portions may advantageously provide an anchoring mechanism for further securing the stent 550 at the treatment site after deployment. In another advantageous feature, the stent embodiment 550 illustrated in **FIG. 35** may be constructed in an inexpensive manner and provides a modular design that may be combined in a variety of different ways to provide an expandable stent suited for a particular purpose.

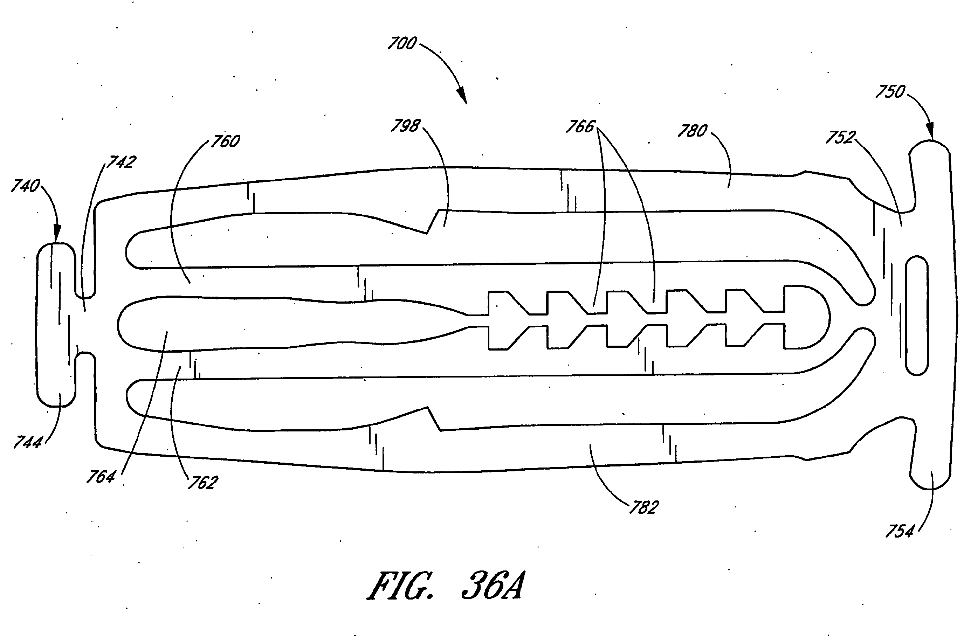

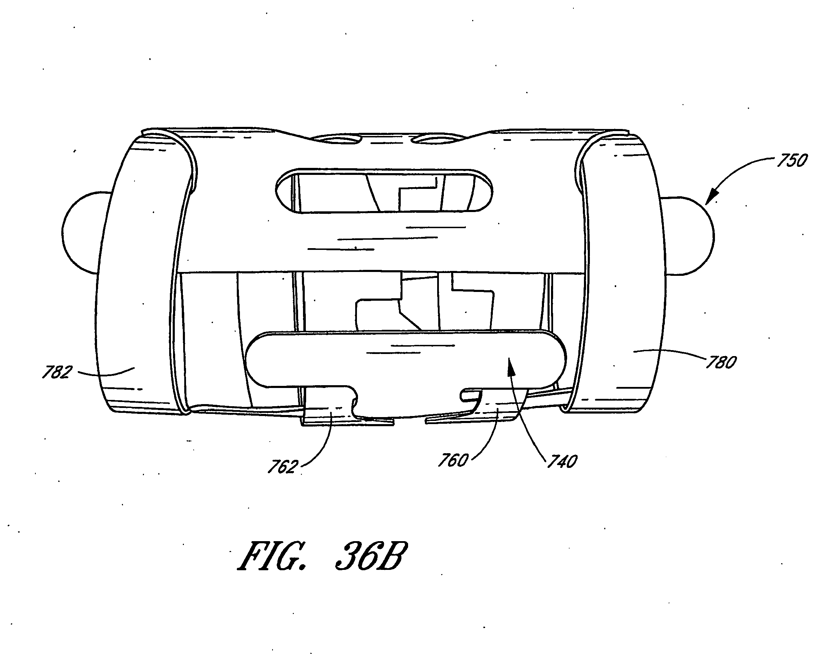

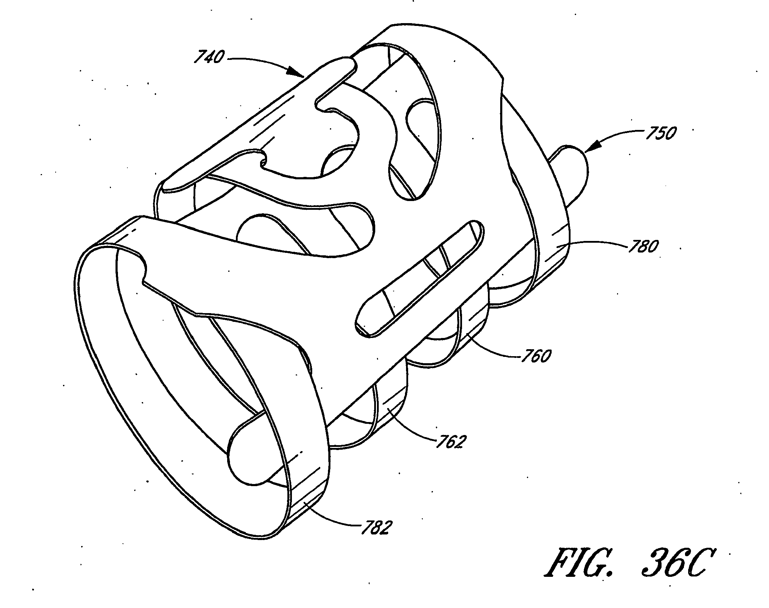

**FIGS. 36A-C** illustrate yet another alternative embodiment of the present invention wherein an expandable stent is formed from a single element 700. The single element 700 may function in a manner similar to certain embodiments described above. More particularly, the element 700 includes a locking tab 740 having a wide head portion 744 and a thin neck portion 742. In embodiments where the stent is fabricated from a shape-memory material (e.g., Nitinol), the stent may optionally include a hold-down tab 750 having a wide head portion 754 and a thin neck portion 752. Still further, the stent includes first and second deflectable members 760, 762 formed with teeth 766 along an inner edge. The element 700 also includes first and second containment members 780, 782 disposed in parallel to the deflectable members. As illustrated in **FIG. 36B****,** the single radial element is rolled onto itself to provide a tubular member with the head portion 742 of the locking tab 740 extending through a gap 764 between the deflectable members 760, 762. When in the collapsed condition, as shown in **FIG. 36B****,** the optional hold-down tab 750 is held within recesses (see element 788 of **FIG. 36A****)** that prevents the stent from expanding during delivery to a treatment site. However, during delivery, the optional hold-down tab 750 may be released from the recesses 788 and the diameter of the radial element expands. During expansion, the locking tab 740 passes through the teeth 766 along the deflectable members 760, 762 until the stent is expanded to the desired diameter, as shown in **FIG. 36C****.** The configurations of the teeth prevent the locking tab 740 from moving back, thereby ensuring that the stent is held in the expanded condition. In an advantageous feature, this embodiment, which has a "jelly-roll" configuration, does not involve any interconnected components and therefore benefits from simplicity in construction. Accordingly, during use, this embodiment provides excellent reliability and structural integrity.

Although a stent formed from a single integral element is described above as having particular mechanical characteristics for locking the stent in the expanded condition, a variety of other "slide-and-lock" mechanisms may be used without departing from the scope of the invention. For example, other suitable locking mechanism may be found in U.S. Patent No. 5,344,426 to Lau, U.S. Patent Nos. 5,735,872 and 5,876,419 to Carpenter, U.S. Pat. No. 5,741,293 to Wijay, U.S. Patent No. 5,984,963 to Ryan, U.S. Patent Nos. 5,441,515 and 5,618,299 by Khosravi, U.S. Patent No. 5,306,286 to Stack, U.S. Patent No. 5,443,500 to Sigwart, U.S. Patent No. 5,449,382 to Dayton, U.S. Patent No. 6,409,752 to Batman, and the like. In addition, many of the slide-and-lock mechanisms disclosed in the above patents may be suitable for use with stents embodiments comprising slidable interconnected elements of the type described above.

Although certain preferred embodiments are described above as providing mono-directional expansion during stent deployment, it will be appreciated that, in another mode of the present invention, the teeth or other engaging elements may be shaped and positioned to allow bi-directional movement (i.e., both expansion and contraction). More particularly, the teeth may be constructed to allow for two-way movement between adjacent radial elements, such that the stent diameter may be collapsed after deployment. The teeth create a barrier that resists the stent from expanding or reducing in diameter. However, the resistance created by the teeth may be overcome during placement of the stent on a balloon and during deployment in the vessel. Preferably, the amount of resistance created by the teeth is selected such that the stent diameter will not reduce due to external pressures after deployment in the vessel. However, the teeth do not provide a locking mechanism that limits stent movement to mono-directional expansion. Accordingly, the diameter of the stent may be reduced for placement on an expandable member. This feature provides a constraining or "hold-down" mechanism that allows the stent to be placed on expandable member and also prevents the stent from expanding prematurely. This embodiment advantageously obviates the need for deformable tabs, pins, crimping mechanisms or other hold-down mechanisms.

### Metal Stents and Methods of manufacturing

Preferred materials for making the stents in accordance with some embodiments of the invention include cobalt chrome, 316 stainless steel, tantalum, titanium, tungsten, gold, platinum, iridium, rhodium and alloys thereof or pyrolytic carbon. In still other alternative embodiments, the stents may be formed of a corrodible material, for instance, a magnesium alloy. Although preferred stent embodiments have been described as being conventional balloon expandable stents, those skilled in the art will appreciate that stent constructions according to the present invention may also be formed from a variety of other materials to make a stent crush-recoverable. For example, in alternative embodiments, such as self expandable stents, shape memory alloys that allow for such as Nitinol and Elastinite® may be used in accordance with embodiments of the invention.

Preferably, sheets are work-hardened prior to forming of the individual stent elements to increase strength. Methods of work hardening are well known in the art. Sheets are rolled under tension, annealed under heat and then re-worked. This may be continued until the desired modulus of hardness is obtained. Most stents in commercial use today employ 0% to 10% work hardened material in order to allow for "softer" material to deform to a larger diameter. In contrast, because expansion of the sliding and locking radial elements in accordance with embodiments of the invention depends on sliding rather than material deformation, it is preferred to use harder materials, preferably in the range of about 25-95% work hardened material to allow for thinner stent thickness. More preferably, the stent materials are 50-90% work hardened and most preferably, the materials are 80-85% work hardened.

Preferred methods of forming the individual elements from the metal sheets may be laser cutting, laser ablation, die-cutting, chemical etching, plasma etching and stamping and water jet cutting of either tube or flat sheet material or other methods known in the art which are capable of producing high-resolution components. The method of manufacture, in some embodiments, depends on the material used to form the stent. Chemical etching provides high-resolution components at relatively low price, particularly in comparison to high cost of competitive product laser cutting. Some methods allow for different front and back etch artwork, which could result in chamfered edges, which may be desirable to help improve engagements of lockouts. Further one may use plasma etching or other methods known in the art which are capable of producing high-resolution and polished components. The current invention is not limited to the means by which stent or stent elements can be fabricated.

Once the base geometry is achieved, the elements can be assembled numerous ways. Tack-welding, adhesives, mechanical attachment (snap-together and/or weave together), and other art-recognized methods of attachment, may be used to fasten the individual elements. Some methods allow for different front and back etch artwork, which could result in chamfered edges, which may be desirable to help improve engagements of lockouts. In one preferred method of manufacture, the components of the stent may be heat set at various desired curvatures. For example, the stent may be set to have a diameter equal to that of the deflated balloon, as deployed, at a maximum diameter, or greater than the maximum diameter. In yet another example, elements can be electropolished and then assembled, or electropolished, coated, and then assembled, or assembled and then electropolished.

In another embodiment, in particular with shape memory alloys, the stent is heat set at beyond the maximum diameter then built mid diameter than placed over catheter and reverse ratcheted and locked into smaller diameter and onto catheter with positive catch hold down mechanism to achieve a small profile and excellent retention.

### Polymeric Stents

While metal stents possess certain desirable characteristics, the useful lifespan of a stent is estimated to be in the range of about 6 to 9 months, the time at which in-stent restenosis stabilizes and healing plateaus. In contrast to a metal stent, a bioresorbable stent may not outlive its usefulness within the vessel. Moreover, a bioresorbable stent may be used to deliver a greater dose of a therapeutic agent, deliver multiple therapeutic agents at the same time or at various times of its life cycle, to treat specific aspects or events of vascular disease. Additionally, a bioresorbable stent may also allow for repeat treatment of the same approximate region of the blood vessel. Accordingly, there remains an important unmet need to develop temporary (i.e., bioresorbable) and radiopaque stents, wherein the polymeric materials used to fabricate these stents have the desirable qualities of metal (e.g., sufficient radial strength and radiopacity, etc.), while circumventing or alleviating the many disadvantages or limitations associated with the use ofpermanent metal stents.

In one preferred embodiment, the stent may be formed from biocompatible polymers that are bio-resorbable (e.g., bio-erodible or bio-degradable). Bio-resorbable materials are preferably selected from the group consisting of any hydrolytically degradable and/or enzymatically degradable biomaterial. Examples of suitable degradable polymers include, but are not limited to, polyhydroxybutyrate /polyhydroxyvalerate copolymers (PHV/PHB), polyesteramides, polylactic acid, hydroxy acids (i.e. lactide, glycolide, hydroxybutyrate), polyglycolic acid, lactone based polymers, polycaprolactone, poly(propylene fumarate-co-ethylene glycol) copolymer (aka fumarate anhydrides), polyamides, polyanhydride esters, polyanhydrides, polylactic acid/polyglycolic acid with a calcium phosphate glass, polyorthesters, silk-elastin polymers, polyphosphazenes, copolymers of polylactic acid and polyglycolic acid and polycaprolactone, aliphatic polyurethanes, polyhydroxy acids, polyether esters, polyesters, polydepsidpetides, polysaccharides, polyhydroxyalkanoates, and copolymers thereof.

In one mode, the degradable materials are selected from the group consisting of poly(glycolide-trimethylene carbonate), poly(alkylene oxalates), polyaspartimic acid, polyglutarunic acid polymer, poly-p-dioxanone, poly-beta.-dioxanone, asymmetrically 3,6-substituted poly-1,4-dioxane-2,5-diones, polyalkyl-2-eyanoacrylates, polydepsipeptides (glycine-DL-lactide copolymer), polydihydropyranes, polyalkyl-2-cyanoacrylates, poly-.beta.-maleic acid (PMLA), polyalkanotes and poly-.beta.-alkanoic acids. There are many other degradable materials known in the art. (See e.g., Biomaterials Science: An Introduction to Materials in Medicine (29 July, 2004) Ratner, Hoffman, Schoen, and Lemons; and Atala, A., Mooney, D. Synthetic Biodegradable Polymer Scaffolds. 1997 Birkhauser, Boston).

Further still, in a more preferred embodiment, the stents may be formed of a polycarbonate material, such as, for example, tyrosine-derived polycarbonates, tyrosine-derived polyarylates, iodinated and/or brominated tyrosine-derived polycarbonates, iodinated and/or brominated tyrosine-derived polyarylates. For additional information, see U.S. Patent Nos. 5,099,060, 5,198,507, 5,587,507, 5,658,995, 6,048,521, 6,120,491, 6,319,492, 6,475,477, 5,317,077, and 5,216,115. In another preferred embodiment, the polymer is any of the biocompatible, bioabsorbable, radiopaque polymers disclosed in U.S. Patent Application Nos. 60/601,526; 60/586,796; and 10/952,202.

Natural polymers (biopolymers) include any protein or peptide. Preferred biopolymers may be selected from the group consisting of alginate, cellulose and ester, chitosan, collagen, dextran, elastin, fibrin, gelatin, hyaluronic acid, hydroxyapatite, spider silk, cotton, other polypeptides and proteins, and any combinations thereof.

In yet another alternative embodiment, shape-shifting polymers may be used to fabricate stents constructed according to the present invention. Suitable shape-shifting polymers may be selected from the group consisting of polyhydroxy acids, polyorthoesters, polyether esters, polyesters, polyamides, polyesteramides, polydepsidpetides, aliphatic polyurethanes, polysaccharides, polyhydroxyalkanoates, and copolymers thereof For addition disclosure on bio-degradable shape-shifting polymers, see U.S. Patent No. 6;160,084. For additional disclosure on shape memory polymers, see U.S. Patent Nos. 6,388,043 and 6,720,402.
Further the transition temperature may be set such that the stent is in a collapsed condition at a normal body temperature. However, with the application of heat during stent placement and delivery, such as via a hot balloon catheter or a hot liquid (e.g., saline) perfusion system, the stent expands to assume its final diameter in the body lumen. When a thermal memory material is used, it may provide a crush-recoverable structure.

Further still, stents may be formed from biocompatible polymers that are biostable (e.g., non-degrading and non-erodible). Examples of suitable non-degrading materials include, but are not limited to, polyurethane, Delrin, high density polyethylene, polypropylene, and poly(dimethyl siloxane).

In some embodiments, the layers may comprise or contain any example of thermoplastic, such as the following, among others: fluorinated ethylene-propylene, poly(2-hydroxyethlmethacrylate (aka pHEMA), poly(ethylene terephthalate) fiber (aka Dacron®) or film (Mylar®), poly(methyl methacrylate (aka PMMA), Poly(tetraflouroethylene) (aka PTFE and ePTFE and Gore-Tex®), poly(vinylchloride), polyacrylates and polyacrylonitrile (PAN), polyamides (aka Nylon), polycarbonates and polycarbonate urethanes, polyethylene and poly(ethylene-co-vinyl acetate), polypropylene, polypropylene, polystyrene, polysulphone, polyurethane and polyetherurethane elastomers such as Pellethane®) and Estane®**,** Silicone rubbers, Siloxane, polydimethylsiloxane (aka PDMS), Silastic®, Siliconized Polyurethane.

### Methods of Manufacturing and Assembling Polymeric Stents

Where plastic and/or degradable materials are used, the elements may be made using laser ablation with a screen, stencil or mask; solvent casting; forming by stamping, embossing, compression molding, centripetal spin casting and molding; extrusion and cutting, three-dimensional rapid prototyping using solid free-form fabrication technology, stereolithography, selective laser sintering, or the like; etching techniques comprising plasma etching; textile manufacturing methods comprising felting, knitting, or weaving; molding techniques comprising fused deposition modeling, injection molding, room temperature vulcanized molding, or silicone rubber molding; casting techniques comprising casting with solvents, direct shell production casting, investment casting, pressure die casting, resin injection, resin processing electroforming, or injection molding or reaction injection molding. Certain preferred embodiments with the present polymers may be shaped into stents via combinations of two or more thereof, and the like.

Such processes may further include two-dimensional methods of fabrication such as cutting extruded sheets of polymer, via laser cutting, etching, mechanical cutting, or other methods, and assembling the resulting cut portions into stents, or similar methods of three-dimensional fabrication of devices from solid forms. For additional information, see U.S. Patent Application No. 10/655,338.

Stents of the preferred embodiment are manufactured with elements prepared in full stent lengths or in partial lengths of which two or more are then connected or attached. If using partial lengths, two or more may be connected or attached to comprise a full length stent. In this arrangement the parts are assembled to give rise to a central opening. The assembled full or partial length parts and/or modules may be assembled by inter-weaving them in various states, from a collapsed state, to a partially expanded state, to an expanded state.

Further, elements may be connected or attached by solvent or thermal bonding, or by mechanical attachment If bonding, preferred methods of bonding comprise the use of ultrasonic radiofrequency or other thermal methods, and by solvents or adhesives or ultraviolet curing processes or photoreactive processes. The elements may be rolled by thermal forming, cold forming, solvent weakening forming and evaporation, or by preforming parts before linking.

Another method of manufacture allows for assembly of the stent components that have been cut out and assembled into flat series of radial elements. The linkage elements between longitudinally adjacent series of radial elements may be connected (e.g., by welding, inter-weaving frame elements, etc.), the flat sheets of material are rolled to form a tubular member. Coupling arms from floating coupling elements and end portions may be joined (e.g., by welding) to maintain the tubular shape. In embodiments that do not include coupling elements, the end portions of the top and bottom radial elements in a series may be joined. Alternatively, where sliding is desired throughout the entire circumference, a sliding and locking articulation can be made between the end portion of the top radial element and the rib(s)/rails of the bottom radial element (e.g., by tack-welding, heat-staking or snap-together). Similarly, a corresponding articulation can be made between the end portion of the bottom radial element and the rib(s)/rails of the top radial element.

Rolling of the flat series of module(s) to form a tubular member can be accomplished by any means known in the art, including rolling between two plates, which are each padded on the side in contact with the stent elements. One plate is held immobile and the other can move laterally with respect to the other. Thus, the stent elements sandwiched between the plates may be rolled about a mandrel by the movement of the plates relative to one another. Alternatively, 3-way spindle methods known in the art may also be used to roll the tubular member. Other rolling methods that may be used in accordance with the present invention include those used for "jelly-roll" designs, as disclosed for example, in U.S. Pat. Nos. 5,421,955, 5,441,515, 5,618,299, 5,443,500, 5,649,977, 5,643,314 and 5,735,872.

The construction of the slide-and-lock stents in these fashions provides a great deal of benefit over the prior art. The construction of the locking mechanism is largely material-independent. This allows the structure of the stent to comprise high strength materials, not possible with designs that require deformation of the material to complete the locking mechanism. The incorporation of these materials will allow the thickness required of the material to decrease, while retaining the strength characteristics of thicker stents. In preferred embodiments, the frequency of catches, stops or teeth present on selected circumferential elements prevents unnecessary recoil of the stent subsequent to expansion.

### Radiopacity

Traditional methods for adding radiopacity to a medical product include the use of metal bands, inserts and/or markers, electrochemical deposition (i.e., electroplating), or coatings. The addition of radiopacifiers (i.e., radiopaque materials) to facilitate tracking and positioning of the stent could be accommodated by adding such an element in any fabrication method, by absorbing into or spraying onto the surface of part or all of the device. The degree of radiopacity contrast can be altered by element content.

For plastics and coatings, radiopacity may be imparted by use of monomers or polymers comprising iodine or other radiopaque elements, i.e., inherently radiopaque materials. Common radiopaque materials include barium sulfate, bismuth subcarbonate, and zirconium dioxide. Other radiopaque elements include: cadmium, tungsten, gold, tantalum, bismuth, platium, iridium, and rhodium. In one preferred embodiment, a halogen such as iodine and/or bromine may be employed for its radiopacity and antimicrobial properties.

### Multi-Material Vascular Prosthesis

In still other alternative embodiments, various materials (e.g., metals, polymers, ceramics, and therapeutic agents) may be used to fabricate stent embodiments. The embodiments may comprise: 1) differentially layered materials (through the vertical or radial axis) to create a stack of materials (materials may be stacked in any configuration, e.g., parallel, staggered, etc.); 2) spatially localized materials which may vary along the long axis and/or thickness of the stent body; 3) materials that are mixed or fused to create a composite stent body; 4) embodiments whereby a material is laminated (or coated) on the surface of the stent body (see Stent Surface Coatings with Functional Properties as well as see Therapeutic Agents Delivered by Stents); and, 5) stents comprised of 2 or more parts where at least one part is materially distinct from a second part, or any combination thereof.

The fashioning of a slide-and-lock multi-material stent can have between two or more materials. Thickness of each material may vary relative to other materials. This approach as needed or desired allows an overall structural member to be built with each material having one or more functions contributing towards enabling prosthesis function which includes, but is not limited to: 1) enabling mechanical properties for stent performance as defined by ultimate tensile strength, yield strength, Young's modulus, elongation at yield, elongation at break, and Poisson's ratio; 2) enabling the thickness of the substrate, geometrical shape (e.g., bifurcated, variable surface coverage); 3) enabling chemical properties of the material that bear relevance to the materials performance and physical state such as rate of degradation and resorption (which may impact therapeutic delivery), glass transition temperature, melting temperature, molecular weight; 4) enabling radiopacity or other forms of visibility and detection; 5) enabling radiation emission; 6) enabling delivery of a therapeutic agent (see Therapeutic Agents Delivered by Stents); and 7) enabling stent retention and/or other functional properties (see Stent Surface Coatings with Functional Properties).

In some embodiments, the materials may comprise load-bearing properties, elastomeric properties, mechanical strength that is specific to a direction or orientation e.g., parallel to another material and/or to the long axis of the stent, or perpendicular or uniform strength to another material and/or stent. The materials may comprise stiffeners, such as the following, boron or carbon fibers, pyrolytic carbon. Further, stents may be comprised of at least one re-inforcement such a fibers, nanoparticles or the like.

In another preferred mode of the invention, the stent is made, at least in part, from a polymeric material, which may be degradable. The motivation for using a degradable stent is that the mechanical support of a stent may only be necessary for several weeks. In some embodiments, bioresorbable materials with varying rates of resorption may be employed. For additional information, see U.S. Patent Application No. 10/952,202 and 60/601,526. Degradable polymeric stent materials may be particularly useful if it also controls restenosis and thrombosis by delivering pharmacologic agents. Degradable materials are well suited for therapeutic delivery (see Therapeutic Agents Delivered by Stents).

In some embodiments, the materials may comprise or contain any class of degradable polymer as previously defined. Along with variation in the time of degradation and/or resorption the degradable polymer may have other qualities that are desirable. For example, in some embodiments the materials may comprise or contain any example of natural polymers (biopolymers) and/or those that degrade by hydrolytic and/or enzymatic action. In some embodiments, the material may comprise or contain any example of hydrogels that may or may not be thermally reversible hydrogels, or any example of a light or energy curable material, or magnetically stimulateable (responding) material. Each of these responses may provide for a specific functionality.

In some embodiments, the materials may comprise or be made from or with constituents which has some radiopaque material alternatively, a clinically visible material which is visible by x-ray, fluoroscopy, ultrasound, MRI, or Imatron Electron Beam Tomography (EBT).

In some embodiments, one or more of the materials may emit predetermined or prescribed levels of therapeutic radiation. In one embodiment, the material can be charged with beta radiation. In another embodiment, the material can be charged with Gamma radiation. In yet another embodiment, the material can be charged with a combination of both Beta and Gamma radiation. Stent radioisotopes that may be used include, but are not limited to, 103Pd and 32P (phosphorus-32) and two neutron-activated examples, 65Cu and 87Rb20, (90)Sr, tungsten-188 (188).

In some embodiments, one or more of the materials may comprise or contain a therapeutic agents The therapeutic agents may have unique, delivery kinetics, mode of action, dose, half-life, purpose, et cetera. In some embodiments, one or more of the materials comprise an agent which provides a mode and site of action for therapy for example by a mode of action in the extracellular space, cell membrane, cytoplasm, nucleus and/or other intracellular organelle. Additionally an agent that serves as a chemoattractant for specific cell types to influence tissue formation and cellular responses for example host-biomaterial interactions, including anti-cancer effects. In some embodiments, one or more of the materials deliver cells in any form or state of development or origin. These could for example be encapsulated in a degradable microsphere, or mixed directly with polymer, or hydrogel and serve as vehicle for pharmaceutical delivery. Living cells could be used to continuously deliver pharmaceutical type molecules, for instance, cytokines and growth factors. Nonliving cells may serve as a limited release system. For additional concepts of therapeutic delivery, see the section entitled: Therapeutic Agents Delivered by Stents.

### Therapeutic Agents Delivered by Stents

In another preferred variation, the stent further comprises an amount of a therapeutic agent (as previously defined for a pharmaceutical agent and/or a biologic agent) sufficient to exert a selected therapeutic effect. In some preferred embodiments of the stent (e.g., polymer stents and multi-material stents) the therapeutic agent is contained within the stent as the agent is blended with the polymer or admixed by other means known to those skilled in the art. In other preferred embodiments of the stent, the therapeutic agent is delivered from a polymer coating on the stent surface. In some preferred embodiments of the stent a therapeutic agent is localized in or around a specific structural aspect of the device.

In another preferred variation the therapeutic agent is delivered by means of a non-polymer coating. In other preferred embodiments of the stent, the therapeutic agent is delivered from at least one region or one surface of the stent. The therapeutic can be chemically bonded to the polymer or carrier used for delivery of the therapeutic from at least one portion of the stent and/or the therapeutic can be chemically bonded to the polymer that comprises at least one portion of the stent body. In one preferred embodiment, more than one therapeutic agent may be delivered.

The amount of the therapeutic agent is preferably sufficient to inhibit restenosis or thrombosis or to affect some other state of the stented tissue, for instance, heal a vulnerable plaque, and/or prevent rupture or stimulate endothelialization or limit other cell types from proliferating and from producing and depositing extracellular matrix molecules. The agent(s) may be selected from the group consisting of antiproliferative agents, antiinflammatory, anti-matrix metalloproteinase, and lipid lowering, cholesterol modifying, antithrombotic and antiplatelet agents, in accordance with preferred embodiments of the present invention. Some of these preferred anti-proliferative agents that improve vascular patency include without limitation paclitaxel, Rapamycin, ABT-578, everolimus, dexamethasone, nitric oxide modulating molecules for endothelial function, tacrolimus, estradiol, mycophenolic acid, C6-ceramide, actinomycin-D and epothilones, and derivatives and analogs of each.

Some of these preferred agents act as an antiplatelet agent, antithrombin agent, compounds to address other pathologic events and/or vascular diseases. Various therapeutic agents may be classified in terms of their sites of action in the host: agents that exert their actions extracellularly or at specific membrane receptor sites, those that act on the plasma membrane, within the cytoplasm, and/or the nucleus.

In addition to the aforementioned, therapeutic agents may include other pharmaceutical and/or biologic agents intended for purposes of treating body lumens other than arteries and/or veins). Therapeutic agents may be specific for treating nonvascular body lumens such as digestive lumens (e.g., gastrointestinal, duodenum and esophagus, biliary ducts), respiratory lumens (e.g., tracheal and bronchial), and urinary lumens (e.g., urethra). Additionally such embodiments may be useful in lumens of other body systems such as the reproductive, endocrine, hematopoietic and/or the integumentary, musculoskeletal/orthopedic and nervous systems (including auditory and ophthalmic applications); and finally, stent embodiments with therapeutic agents may be useful for expanding an obstructed lumen and for inducing an obstruction (e.g., as in the case of aneurysms).

Therapeutic release may occur by controlled release mechanisms, diffusion, interaction with another agent(s) delivered by intravenous injection, aerosolization, or orally. Release may also occur by application of a magnetic field, an electrical field, or use of ultrasound.

### Stent Surface Coatings with Functional Properties

In addition to stents that may deliver a therapeutic agent, for instance delivery of a biological polymer on the stent such as a repellant phosphorylcholine, the stent may be coated with other bioresorbable polymers predetermined to promote biological responses in the body lumen desired for certain clinical effectiveness. Further the coating may be used to mask (temporarily or permanently) the surface properties of the polymer used to comprise the stent embodiment. The coating may be selected from the broad class of any biocompatible bioresorbable polymer which may include any one or combination of halogenated and/or non-halogenated which may or may not comprise any poly(alkylene glycol). These polymers may include compositional variations including homopolymers and heteropolymers, stereoisomers and/or a blend of such polymers. These polymers may include for example, but are not limited to, polycarbonates, polyarylates, poly(ester amides), poly(amide carbonates), trimethylene carbonate, polycaprolactone, polydioxane, polyhydroxybutyrate, poly-hydroxyvalerate, polyglycolide, polylactides and stereoisomers and copolymers thereof, such as glycolide/lactide copolymers. In a preferred embodiment, the stent is coated with a polymer that exhibits a negative charge that repels the negatively charged red blood cells' outer membranes thereby reducing the risk of clot formation. In another preferred embodiment, the stent is coated with a polymer that exhibits an affinity for cells, (e.g., endothelial cells) to promote healing. In yet another preferred embodiment, the stent is coated with a polymer that repels the attachment and/or proliferation of specific cells, for instance arterial fibroblasts and/or smooth muscle cells in order to lessen restenosis and/or inflammatory cells such as macrophages.

Described above are the stents of the present invention that may be modified with a coating to achieve functional properties that support biological responses. Such coatings or compositions of material with a therapeutic agent may be formed on stents or applied in the process of making a stent body via techniques such as dipping, spray coating, cross-linking combinations thereof, and the like. Such coatings or compositions of material may also serve purpose other than delivering a therapeutic, such as to enhance stent retention on a balloon when the coating is placed intraluminally on the stent body and/or placed over the entire device after the stent is mounted on the balloon system to keep the stent in a collapsed formation. Other purposes can be envisioned by those skilled in the art when using any polymer material.

In one aspect of the invention, a stent would have a coating applied that has specific mechanical properties. The properties may include *inter alia* thickness, tensile strength, glass transition temperature, and surface finish. The coating is preferably applied prior to final crimping or application of the stent to the catheter. The stent may then be applied to the catheter and the system may have either heat or pressure or both applied in a compressive manner. In the process, the coating may form frangible bonds with both the catheter and the other stent surfaces. The bonds would enable a reliable method of creating stent retention and of holding the stent crossing profile over time. The bonds would break upon the balloon deployment pressures. The coating would be a lower Tg than the substrate to ensure no changes in the substrate.

### Stent Deployment

First, a catheter is provided wherein an expandable member, preferably an inflatable balloon, such as an angioplasty balloon, is provided along a distal end portion. One example of a balloon catheter for use with a stent is described in U.S. Patent No. 4,733,665 to Palmaz. A stent on a catheter is commonly collectively referred to as a stent-system. Catheters include but are not limited to over-the-wire catheters, coaxial rapid-exchange designs and the Medtronic Zipper Technology that is a new delivery platform. Such catheters may include for instance those described in Bonzel U.S. Patent Nos. 4,762,129 and 5,232,445 and by Yock U.S. Patent Nos. 4,748,982; 5,496,346; 5,626,600; 5,040,548; 5,061,273; 5,350,395; 5,451,233 and 5,749,888. Additionally, catheters may include for instance those as described in U.S. Patent Nos. 4,762,129; 5,092,877; 5,108,416; 5,197,978; 5,232,445; 5,300,085; 5,445,646; 5,496,275; 5,545,135; 5,545,138; 5,549,556; 5,755,708; 5,769,868; 5,800,393; 5,836,965; 5,989,280; 6,019,785; 6,036,715; 5,242,399; 5,158,548; and 6,007,545.

Catheters may be specialized with highly compliant polymers and for various purposes such as to produce an ultrasound effect, electric field, magnetic field, light and/or temperature effect. Heating catheters may include for example those described in U.S. Patent No. 5,151,100, 5,230,349; 6,447,508; and 6,562,021 as well as WO9014046A1. Infrared light emitting catheters may include for example those described in U. S. Patent Nos. 5,910,816 and 5,423,321.

An expandable member, such as an inflatable balloon, is preferably used to deploy the stent at the treatment site. As the balloon is expanded, the radial force of the balloon overcomes the initial resistance of the constraining mechanism, thereby allowing the stent to expand. As the balloon is inflated, the radial elements slide with respect to each other along the surface of the balloon until the stent has been expanded to a desired diameter.

The stent of embodiments of the invention are adapted for deployment using conventional methods known in the art and employing percutaneous transluminal catheter devices. This includes deployment in a body lumen by means of a balloon expandable design whereby expansion is driven by the balloon expanding. Alternatively, the stent may be mounted onto a catheter that holds the stent as it is delivered through the body lumen and then releases the stent and allows it to self-expand into contact with the body lumen. The restraining means may comprise a removable sheath and/or a mechanical aspect of the stent design.

Some embodiments of the invention may be useful in coronary arteries, carotid arteries, vascular aneurysms (when covered with a sheath), and peripheral arteries and veins (e.g., renal, iliac, femoral, popliteal, subclavian, aorta, intercranial, etc.). Other nonvascular applications include gastrointestinal, duodenum, biliary ducts, esophagus, urethra, reproductive tracts, trachea, and respiratory (e.g., bronchial) ducts. These applications may or may not require a sheath covering the stent.

It is desirable to have the stent radially expand in a uniform manner. Alternatively, the expanded diameter may be variable and determined by the internal diameter and anatomy of the body passageway to be treated. Accordingly, uniform and variable expansion of the stent that is controlled during deployment is not likely to cause a rupture of the body passageway. Furthermore, the stent will resist recoil because the locking means resist sliding of the mating elements. Thus, the expanded intraluminal stent will continue to exert radial pressure outward against the wall of the body passageway and will therefore, not migrate away from the desired location.

From the foregoing description, it will be appreciated that a novel approach for expanding a lumen has been disclosed. While the components, techniques and aspects of the invention have been described with a certain degree of particularity, it is manifest that many changes may be made in the specific designs, constructions and methodology herein above described.

While a number of preferred embodiments of the invention and variations thereof have been described in detail, other modifications and methods of using and medical applications for the same will be apparent to those of skill in the art. Accordingly, it should be understood that various applications, modifications, materials, and substitutions may be made of equivalents.

Various modifications and applications of the invention may occur to those who are skilled in the art. It should be understood that the invention is not limited to the embodiments set forth herein for purposes of exemplification, but is to be defined only by a fair reading of the appended claims, including the full range of equivalency to which each element thereof is entitled.

The stent preferably comprises at least one longitudinal module, which consists of a series of radial elements, including one or more slide-and-lock radial elements and optionally one or more passive radial elements, linked in the longitudinal axis by flexible coupling portions. Preferably, the radial elements from two or more similar longitudinal modules are slidably connected to circumferentially adjacent radial elements. Of course, single module (or jellyroll-type) embodiments are also encompassed within the scope of the present disclosure. Each module is preferably a discrete, unitary structure that does not stretch or otherwise exhibit any substantial permanent deformation during stent deployment.

### References

Some of the references cited herein are listed below:
- Charles R, Sandirasegarane L, Yun J, Bourbon N, Wilson R, Rothstein RP, et al. Ceramide-Coated Balloon Catheters Limit Neointimal Hyperplasia after Stretch Injury in Carotid Arteries. Circ Res 2000;87(4):282-288.
- Coroneos E, Martinez M, McKenna S, Kester M. Differential regulation of sphingomyelinase and ceramidase activities by growth factors and cytokines. Implications for cellular proliferation and differentiation. J Biol Chem 1995;270(40):23305-9.
- Coroneos E, Wang Y, Panuska JR, Templeton DJ, Kester M. Sphingolipid metabolites differentially regulate extracellular signal-regulated kinase and stress-activated protein kinase cascades. Biochem J 1996;316(Pt 1): 13-7.
- Jacobs LS, Kester M. Sphingolipids as mediators of effects of platelet-derived growth factor in vascular smooth muscle cells. Am J Physiol 1993;265(3 Pt 1):C740-7.
- Tanguay JF, Zidar JP, Phillips HR, 3rd, Stack RS. Current status of biodegradable stents. Cardiol Clin 1994;12(4):699-713.
- Nikol S, Huehns TY, Hofling B. Molecular biology and post-angioplasty restenosis. Atherosclerosis 1996;123(1-2):17-31.
- Biomaterials Science: An Introduction to Materials in Medicine (29 July, 2004) Ratner, Hoffman, Schoen, and Lemons

## Claims

1. A slide-and-lock stent (10), comprising a tubular member (1200) having longitudinal and circumferential axes, said tubular member (1200) comprising:
a first radial element (1202) comprising an elongate rail (1204) comprising a plurality of deflectable teeth (1206), and
a second radial element (1202), circumferentially adjacent to the first radial element, and comprising an engagement means configured to slidably engage said elongate rail (1204) of the first radial element (1202) and deflect said deflectable tooth (1206) as the tooth contacts said engagement means; **characterized in that**
said elongate rail (1204) defines upper (1222) and lower portions (1226), said upper portion (1222) tapering from a first thickness to a second thickness along said circumferential axis approaching a distal edge (1220) of said upper portion (1222), said lower portion (1226) tapering from said first thickness to said second thickness along said circumferential axis approaching a distal edge (1224) of said lower portion (1226); and
said engagement means comprising a closed loop that forms a slot (1210), said closed loop (1208) tapering along said circumferential axis to said second thickness approaching a distal edge of said engagement means, said slot (1210) being sized and configured with said elongate rail (1204) being passable therethrough, such that said tubular member achieves expansion in the circumferential axis with reduced recoil.

2. The slide-and-lock stent according to claim 1, wherein the elongate rail (1204) comprises two rail members (70) with a gap (1214) being disposed therebetween.

3. The slide-and-lock stent according to claim 2, wherein the slot (1210) is sized and configured with the elongate rail (1204) being passable therethrough with the rail members (70) the elongate rail (1204) deflecting toward one another into the gap (1214) when engaged by the slot (1210).

4. The slide-and-lock stent according to any of claims 1-3, wherein the engagement means comprise a closed loop (1208) which defines the slot (1210).

5. The slide-and-lock stent according to any of claims 1-4, wherein the second thickness is approximately one-half of the first thickness.

6. The slide-and-lock stent according to any of claims 1-5, wherein the engagement means tapers from a combined thickness of approximately 1 ½ of the first thickness.

7. The slide-and-lock stent according to any of claims 1-6, wherein the first and second radial elements (1202) have a combined maximum thickness of 1 ½ of the first thickness.

8. The slide-and-lock stent according to any of claims 1-7, wherein the first thickness is approximately 0.1016 mm (0.0040 inches).

## Patentansprüche

1. Schiebe- und Arretier-Stent (10), der ein röhrenförmiges Element (1200) mit längsgerichteten peripheren Achsen umfasst, worin das röhrenförmige Element (1200) umfasst:
ein erstes radiales Element (1202), das eine gestreckte Schiene (1204) umfasst, die mehrere ablenkbare Zähne (1206) umfasst, und
ein zweites radiales Element (1202), das an das erste radiale Element umlaufend angrenzt, und ein Eingriffmittel umfasst, das konfiguriert ist, um mit der gestreckten Schiene (1204) des ersten radialen Elements (1202) verschiebbar in Eingriff zu gelangen und den ablenkbaren Zahn (1206) abzulenken, da der Zahn mit dem Eingriffmittel in Kontakt steht; **dadurch gekennzeichnet, dass**
die gestreckte Schiene (1204) obere (1222) und untere Bereiche (1226) definiert, worin der obere Bereich (1222) sich von einer ersten Dicke zu einer zweiten Dicke entlang der umlaufenden Achse verjüngt, die eine distale Kante (1220) des oberen Bereichs (1222) erreicht, worin sich der untere Bereich (1226) von der ersten Dicke zu der zweiten Dicke entlang der umlaufenden Achse verjüngt, die eine distale Kante (1224) des unteren Bereichs (1226) erreicht; und
worin das Eingriffsmittel eine geschlossene Schleife umfasst, die einen Schlitz (1210) bildet, worin sich die geschlossene Schleife (1208) entlang der umlaufenden Achse zu der zweiten Dicke verjüngt, die eine distale Kante des Eingriffsmittels erreicht, worin der Schlitz (1210) so bemessen und konfiguriert ist, dass die gestreckte Schiene (1204) **dadurch** passt, so dass das röhrenförmige Element eine Ausdehnung in der umlaufenden Achse mit verringertem Rückstoß erzielt.

2. Schiebe- und Arretier-Stent nach Anspruch 1, worin die gestreckte Schiene (1204) zwei Schienenelemente (70) mit einer dazwischen angeordneten Lücke (1214) umfasst.

3. Schiebe- und Arretier-Stent nach Anspruch 2, worin der Schlitz (1210) so bemessen und konfiguriert ist, dass die gestreckte Schiene (1204) **dadurch** passt, wobei die Schienenelemente (70) der gestreckten Schiene (1204) aufeinander zu in die Lücke (1214) abgelenkt werden, wenn sie durch den Schlitz (1210) in Eingriff stehen.

4. Schiebe- und Arretier-Stent nach einem der Ansprüche 1-3, worin das Eingriffmittel eine geschlossene Schleife (1208) umfasst, die den Schlitz (1210) definiert.

5. Schiebe- und Arretier-Stent nach einem der Ansprüche 1-4, worin die zweite Dicke ungefähr die Hälfte der ersten Dicke beträgt.

6. Schiebe- und Arretier-Stent nach einem der Ansprüche 1-5, worin sich das Eingriffsmittel von einer kombinierten Dicke von ungefähr 1 ½ der ersten Dicke verjüngt.

7. Schiebe- und Arretier-Stent nach einem der Ansprüche 1-6, worin die ersten und zweiten radialen Elemente (1202) eine kombinierte maximale Dicke von 1 ½ der ersten Dicke aufweisen.

8. Schieb- und Arretier-Stent nach einem der Ansprüche 1-7, worin die erste Dicke ungefähr 0,1016 mm (0,0040 Zoll) beträgt.

## Revendications

1. Un stent à coulissement et verrouillage (10) comprenant un membre tubulaire (1200) comportant des axes longitudinal et circonférentiel, ledit membre tubulaire (1200) comprenant :
un premier élément radial (1202) comprenant un rail allongé (1204) comprenant une pluralité de dents pliables (1206), et
un second élément radial (1202) adjacent par la circonférence au premier élément radial et comprenant un moyen d'engagement configuré pour engager par glissement ledit rail allongé (1204) du premier élément radial (1202) et plier ladite dent pliable (1206) lorsque la dent contacte ledit moyen d'engagement, **caractérisé en ce que**
ledit rail allongé (1204) définit des portions supérieure (1222) et inférieure (1226), ladite portion inférieure (1222) s'effilant d'une première épaisseur à une seconde épaisseur le long dudit axe longitudinal en s'approchant d'un bord distal (1220) de ladite portion supérieure (1222), ladite portion inférieure (1226) s'effilant d'une première épaisseur à une seconde épaisseur le long dudit axe circonférentiel en s'approchant d'un bord distal (1224) de ladite portion inférieure (1226) ;
ledit moyen d'engagement comprenant une boucle fermée formant une fente (1210), ladite boucle fermée (1208) s'effilant le long dudit axe circonférentiel vers ladite seconde épaisseur en s'approchant d'un bord distal dudit moyen d'engagement, ladite fente (1210) étant dimensionnée et configurée pour que ledit rail allongé (1204) passe au travers de ce dernier de sorte que les éléments tubulaires exercent, avec un recul limité, une expansion dans l'axe circonférentiel.

2. Le stent à coulissement et verrouillage selon la revendication 1 dans lequel le rail allongé (1204) comprend deux membres de rail (70) avec un évidement (1214) disposé entre eux.

3. Le stent à coulissement et verrouillage selon la revendication 2 dans lequel la fente (1210) est dimensionnée et configurée pour que le rail allongé (1204) passe au travers, et pour que les membres de rail (70) du rail allongé (1204) se plient l'un vers l'autre dans l'évidement (1204) lorsque engagé dans la fente (1210).

4. Le stent à coulissement et verrouillage selon l'une des revendications 1-3 dans lequel le moyen d'engagement comprend une boucle fermée (1208) qui définit la fente (1210).

5. Le stent à coulissement et verrouillage selon l'une des revendications 1-4 dans lequel la seconde épaisseur est approximativement une moitié de la première épaisseur.

6. Le stent à coulissement et verrouillage selon l'une des revendications 1-5 dans lequel le moyen d'engagement s'effile d'une épaisseur combinée d'approximativement 1 ½ de la première épaisseur.

7. Le stent à coulissement et verrouillage selon l'une des revendications 1-6 dans lequel les premier et second éléments radiaux (1202) ont une épaisseur combinée maximum de 1 ½ de la première épaisseur.

8. Le stent à coulissement et verrouillage selon l'une des revendications 1-7 dans lequel la première épaisseur est approximativement 0.1.16 mm (0.004 pouce).
